# EUROPEAN PATENT SPECIFICATION

(11) **EP 4 359 800 B1**
(45) Date of publication and mention of the grant of the patent: **03.06.2026**
(21) Application number: 22828869.2
(22) Date of filing: 21.06.2022
(51) Int. Cl.: G01N 33/575

(54) **PREDICTING CANCER RELAPSE**
VORHERSAGE EINES KREBSREZIDIVS
PRÉDICTION DE RECHUTE DE CANCER

(30) Priority: 23.06.2021 US 202163213791 P
(43) Date of publication of application: 01.05.2024
(73) Proprietor: AroCell AB, 168 67 Bromma (SE)
(72) Inventor: ERIKSSON, Staffan, 181 46 Lidingö (SE); VENGE, Per, 753 12 Uppsala (SE)
(74) Representative: Barker Brettell Sweden AB
(86) International application number: PCT/SE2022/050614
(87) International publication number: WO 2022/271069

(56) References cited:
- WO-A1-2015/094106
- WO-A1-2019/201901
- WO-A1-2020/043868
- US-A1- 2008 187 909
- O'NEILL K L ET AL: "Thymidine kinase 1 - A prognostic and diagnostic indicator in ALL and AML patients", BLOOD CANCER JOURNAL, NATURE PUBLISHING GROUP UK, LONDON, vol. 21, no. 3, 25 January 2007 (2007-01-25), pages 560 - 563, XP037783199, ISSN: 0887-6924, [retrieved on 20070125], DOI: 10.1038/SJ.LEU.2404536

## Description

### TECHNICAL FIELD

The present invention generally relates to measurement of thymidine kinase 1 (TK1), and in particular to predicting cancer relapse based on measured serum TK1 levels.

### BACKGROUND

Thymidine kinase 1 (TK1) (EC 2.7.1.21), also referred to as 2'-deocythymidine kinase or ATP-thymidine 5'-phosphotransferase, is an enzyme involved in deoxyribonucleic acid (DNA) precursor synthesis. TK1 phosphorylates thymidine to allow incorporation into DNA. Expression of TK1 is a marker of active cellular proliferation with low intracellular concentration during GO/G1 phase of the cell cycle, and increasing during the S/G2 phases.

A form of TK1 is present at higher levels in sera and plasma from humans and animals with malignant tumors. Therefore, serum TK1 activity measurements have been used for monitoring and for prognostic purpose in several different malignant diseases, but so far primarily in case of leukemia and lymphoma.

Furthermore, TK1 is the only proliferation marker that can be determined in the blood and it is likely to provide a large clinical benefit if available as a routine laboratory test.

The serum TK1 activity has been measured using a radioactive substrate ¹²⁵I-dUrd (the PROLIFIGEN^{®} TK-REA, DiaSorin Inc.) for several decades, but this radio-enzymatic assay has had limited use and preferentially in case of malignant hematologic malignancies. A non-radiometric TK1 activity assay (TK LIAISON^{®} assay, DiaSorin Inc.) has been available since 2000. This is a sensitive and robust assay and has provided clinically valuable information in humans and dogs, particularly for monitoring therapy and predicting relapse.

During the last 15 years antibodies against human TK1 have been available and enabled the determination of the TK1 protein levels, in contrast to TK1 activity, both in hematologic as well as in solid tumor diseases, such as breast carcinomas, and several other forms of solid and hematologic tumors.

One type of TK1 protein determination relies on a dot blot procedure based on anti-TK1 antibodies produced against the C-terminal part of TK1. The main reason for choosing this strategy for antibody production is that the C-terminal region is involved in the cell cycle regulation of TK1. It contains a recognition sequence for initiating the degradation of TK1 during mitosis and it has been assumed that this is an exposed region to which it could be possible to generate antibodies. Although the dot blot assay has been used successfully in a number of studies, a major limitation is that it is not a routine method in clinical laboratory practice.

AroCell TK 210 ELISA is a quantitative immunoassay kit for the determination of TK1 in human blood. The ELISA format is simple and robust, requires no special instrumentation to perform and can easily be incorporated into standard laboratory processes. The AroCell TK 210 ELISA is not only a fast and simple way to monitor TK1 from blood samples but delivers reproducible results you can trust using standard equipment in clinical chemistry.

O'Neill et al., Thymidine kinase 1 - A prognostic and diagnostic indicator in ALL and AML patients, Leukemia (2007) 21: 560-563 discloses that mean serum TK1 levels of ALL patients were significantly different as compared to healthy individuals.

WO 2020/043868 A1 discloses that TK1 represents a valuable biomarker in a method for determining a Prognostic Index (PI) for risk stratification of a patient with aggressive B-cell lymphoma, especially diffuse large B-cell lymphoma (DLBCL).

### SUMMARY

It is a general objective of the invention to accurately predict cancer survival and/or cancer relapse in patients.

This and other objectives are met by embodiments as disclosed herein

An aspect of the invention relates to a method for predicting cancer relapse. The method comprises determining a level of serum thymidine kinase 1 (STK1) material in a body sample from a patient diagnosed with hematological cancer using an antibody or a fragment thereof specifically binding to a serum form of human TK1. The method also comprises selecting a threshold value based on an age of the patient and comparing the determined level of STK1 material with the threshold value selected based on the age of the patient. The method further comprises predicting cancer relapse of the patient based on the comparison.

The present invention predicts cancer relapse based on a comparison between measured STK1 levels an age-dependent threshold value. Experimental data as presented herein indicates that the accuracy when using STK1 as biomarker for hematological cancers will be significantly enhanced when using age-related reference or threshold ranges for patient groups of different ages.

### BRIEF DESCRIPTION OF THE DRAWINGS

The embodiments, together with further objects and advantages thereof, may best be understood by making reference to the following description taken together with the accompanying drawings, in which:
Fig. 1 TK1 concentration in serum before treatment in relation to (1a) Ann Arbor stage and (1b) age adjusted International prognostic index.
Fig. 2 TK1 concentration in serum before treatment in relation to (2a) time to disease recurrence and (2b) survival time.
Fig. 3 difference in (3a) overall survival, (3b) disease free survival and (3c) disease specific survival between patients with high versus low TK1 concentration in serum obtained before treatment. Cut-off for dichotomizing was set to upper limits for healthy individuals, i.e., 0.45 µg/L.
Fig. 4 multivariate analyses of dichotomized TK1 before treatment (cut off at upper normal value of 0.45 µg/L) and aalPI score (4a, 4b) and IPI score (4c, 4d), respectively.
Fig. 5 TK1 concentration measured in blood samples collected within the U-CAN research project. Blood samples during treatment (after third chemotherapy cycle) was not included at launch of the U CAN project, but was added to the U-CAN protocol more recently.
Fig. 6 comparison of TK1 concentration in serum before and after treatment for living and diseased patients.
Fig. 7 TK1 concentration in serum for individuals with age adjusted International Prognostic Index (7a) (aalPI) 0-1 and (7b) aalPI 2-3. Blood samples analyzed were obtained within the U-CAN research project which states blood samples before treatment start, in the middle of the treatment (i.e., after 3 chemotherapy cycles) and after treatment end.
Fig. 8 blood samples during treatment were not obtained at launch but were added to the protocol more recently (samples after third chemotherapy cycle). Number of patients with available TK1 values during treatment are thus limited which is reflected in available ratios of TK1 before/during treatment.
Fig. 9 are Kaplan-Meier plots of probability of disease relapse in relation to TK1 serum concentrations at diagnosis and before treatment in patients with in Diffuse Large B-cell Lymphoma with the cut-off of (A) 0.25 µg/L, (B) 0.35 µg/L, (C) 0.45 µg/L, (D) 0.75 µg/L, (E) 1.05 µg/L and (F) 2.5 µg/L in relation to time of relapse, days.
Fig. 10 are Kaplan-Meier plots of probability of disease relapse in relation to TK1 serum concentrations at diagnosis and before treatment in patients with in Diffuse Large B-cell Lymphoma with the cut-off of 0.45 µg/L in relation to time of relapse, days. (A) all patients, (B) age >66 years and (C) age<67 years.

### DETAILED DESCRIPTION

The present invention generally relates to measurement of thymidine kinase 1 (TK1) and in particular to predicting cancer relapse based on measured serum TK1 (STK1) levels.

The present invention predicts cancer relapse based on a comparison between the measured STK1 levels and an age-dependent threshold value. Experimental data as presented herein indicates that higher concentrations of STK1 correlated with more advanced stages of diffuse large B-cell lymphoma (DLBCL), as an example of a hematological cancer, and that an elevated STK1 level before treatment correlated with shorter disease-fee and disease-specific survival for the full patient cohort. However, when dividing the patient cohort into groups dependent on the age of the patient at the time of diagnosis a different picture arose. For the elderly patients, there was no proportional correlation between the determined STK1 level and the risk for cancer relapse. In clear contrast, already a comparatively low increase in STK1 level above the average level of healthy subjects was associated with increased risk of cancer relapse. Furthermore, the risk for cancer relapse was not significantly different for an elderly patient having a STK1 level slightly above a defined threshold as compared to another elderly patient having a STK1 level much over the defined threshold. The situation was, however, different for younger DLBCL patients, for which comparatively higher STK1 levels were correlated with increased risk of cancer relapse as compared to the elderly DLBCL patients.

Hence, the accuracy when using STK1 as biomarker for hematological cancers will be significantly enhanced when using age-related reference or threshold ranges for patient groups of different ages.

An aspect of the invention relates to a method for predicting cancer relapse. The method comprises determining a level of serum thymidine kinase 1 (STK1) material in a body sample from a patient diagnosed with hematological cancer using an antibody or a fragment thereof specifically binding to a serum form of human TK1. The method also comprises selecting a threshold value based on an age of the patient and comparing the determined level of STK1 material with the threshold value selected based on the age of the patient. The method further comprises predicting cancer relapse of the patient based on the comparison.

TK1 proteins in humans are present in various forms depending on the presence of certain molecules, e.g., presence or absence of adenosine triphosphate (ATP); depending on the concentration of the protein, i.e., high or low concentration; depending on the type of the protein, i.e., native or recombinant TK1; and depending on the site of the protein, i.e., in serum or cytoplasm.

Generally, cytosolic and recombinant human TK1 occurs as tetramers in the presence of ATP or at high concentration, and as dimers in the absence of ATP or at low concentration. The tetramer form of cytosolic and recombinant human TK1 has high TK1 activity whereas the dimer form has lower TK1 activity. Cytosolic TK1, also referred to as cellular TK1, is TK1 present inside cells and can be isolated from such cells.

Human STK1, in clear contrast, can be in the form of high molecular weight complexes, such as oligomers or comprising such oligomers, having TK1 activity and dimer and tetramer forms having very low or even lacking TK1 activity. The oligomerization seems to be related to the formation of disulfide cross linking occurring in the blood. STK1 is found in the blood of a patient and can thereby be determined in, among others, a blood sample, a plasma sample or a serum sample.

STK1 material as used herein refers to STK1 in its various forms, such as dimers, tetramers, oligomers and complexes comprising STK1. The STK1 material is a STK1 material present in blood, blood plasma or serum in patient. The STK1 material may then comprise STK1 in the above-mentioned forms, such as dimers, tetramers, oligomers and complexes comprising STK1. STK1 material also includes complexes with at least one TK1 protein unit and other molecules and/or macromolecules.

In the art, various gene expressions arrays have been proposed to determine the TK1 messenger ribonucleic acid (mRNA) transcripts in cancer cell samples and biopsies, including a lymph node biopsy samples. As mentioned above, TK1 is available in various forms in subjects, including cytosolic TK1 and serum TK1. Gene expression arrays determining TK1 mRNA transcripts from such biopsy samples are mainly assaying TK1 mRNA transcripts of cytosolic TK1 present in cancer cells. Hence, such gene expression arrays cannot be used to determine the level of STK1 material in a subject.

In an embodiment, determining the level of STK1 material comprises contacting the body sample with the antibody or the fragment thereof. This embodiment also comprises measuring an amount of the antibody or the fragment thereof bound to the STK1 material.

Contacting the body sample with the antibody or the fragment thereof may be achieved by adding the antibody or the fragment thereof to the body sample and incubating the body sample with the antibody or the fragment thereof. The antibody or the fragment thereof thereby binds to the STK1 material forming a complex between the antibody or the fragment thereof and the STK1 material. In such an embodiment, measuring the amount of the antibody or the fragment thereof bound to the STK1 material can include measuring or quantifying the complex between the antibody or the fragment thereof and the STK1 material to thereby measure or quantify the amount of the antibody or the fragment thereof bound to the STK1 material.

In an embodiment, the method also comprises correlating the measured amount of the antibody or the fragment thereof bound to the STK1 material to a level of STK1 material. This may be performed using a pre-defined correlation between measured amount of the antibody or the fragment thereof bound to a reference TK1 material and concentration of the reference TK1 material. A typical reference TK1 material that can be used when generating such a pre-defined correlation is recombinant human TK1.

The pre-defined correlation may, thus, be generated by adding the antibody or the fragment thereof to different samples comprising different concentrations of the reference TK1 material, preferably recombinant human TK1. The amount of the antibody or the fragment thereof bound to the reference TK1 material, preferably recombinant human TK1, is then measured in the different samples to thereby get a standard curve, function or relationship between concentration of reference TK1 material, preferably recombinant human TK1, and the measured amount of the antibody or the fragment thereof bound to the reference TK1 material, preferably recombinant human TK1.

This pre-defined correlation, such as standard curve, function or relationship, can then be used to map or convert the measured amount of the antibody or the fragment thereof bound to the STK1 material in the body sample to a concentration of the STK1 material in the body sample.

It is generally preferred if the same type of antibody or fragment thereof is used for generating the pre-defined correlation as for determining a level of STK1 material in a body sample from the patient diagnosed with hematological cancer. Hence, in a preferred embodiment, the antibody or the fragment thereof is capable of specifically binding to not only the serum form of human TK1 but also to the reference TK1 material, preferably recombinant human TK1.

In an embodiment, the body sample is processed prior to or during the incubation of the body sample with the antibody or the fragment thereof. This sample processing may be used to stabilize selected STK1 forms in the body sample and/or to break larger STK1 complexes or oligomers into smaller complexes or multimers.

Hence, in an embodiment, a sample dilution or pretreatment buffer is added to the body sample prior to or in connection with adding the antibody or the fragment thereof to the body sample, preferably prior to adding the antibody or the fragment thereof to the body sample.

In an embodiment, the sample dilution buffer comprises ATP, preferably in a concentration selected within an interval of from 0.5 mM up to 50 mM, such as from 0.5 mM up to 20 mM or from 1.5 mM up to 50 mM. As previously described herein, ATP stabilizes the tetramer form of TK1, which has high enzymatic TK1 activity.

In another embodiment, the sample dilution buffer comprises a reducing agent. The reducing agent may then break disulfide cross links in larger STK1 complexes and oligomers to obtain smaller STK1 forms, such as tetramers. Various reducing agents capable of breaking disulfide bonds can be used according to the embodiments including, but not limited to, dithioerythritol (DTE), dithiothreitol (DTT), dithiobutylamin (DTBA), tris(2-carboxyethyl)phosphine) (TCEP), and combinations thereof. The amount of the reducing agent is typically selected within an interval of from 0.1 mM up to 10 mM.

The sample dilution buffer may, in an embodiment, comprise both ATP and a reducing agent.

The level of STK1 material is, in an embodiment, determined using the antibody or the fragment thereof specifically binding to the serum form of human TK1 in the body sample taken from the patient in connection with diagnosing the patient with hematological cancer or at least prior to start of treatment of the hematological cancer. Hence, in a preferred embodiment, the body sample is preferably taken at the time of diagnosis or at least shortly following the point in time at which the patient was diagnosed with hematological cancer or at least suspected to suffer from hematological cancer.

In an embodiment, the method comprises estimating a hazard ratio (HR) for the patient based on the comparison between the determined level of STK1 material with the selected threshold value. In such an embodiment, predicting cancer relapse of the patient comprises predicting cancer relapse of the patient based on the estimated HR.

In an embodiment, predicting cancer relapse comprises predicting a high risk for cancer relapse of the patient if the determined level of STK1 material in the body sample exceeds the selected threshold value and otherwise predicting a low risk for cancer relapse of the patient.

According to the invention, the method also comprises selecting the threshold value based on the age of the patient.

In a particular embodiment, the method comprises selecting a first threshold value if the age of the patient is equal to or above a defined age and selecting a second, different threshold value if the age of the patient is below the defined age.

Hence, in this particular embodiment, two different age-optimized threshold values are employed and the particular age-optimized threshold value to use is dependent on whether the age of the patient is equal to or above the defined age or below the defined age.

In an embodiment, the second, different threshold value is higher than the first threshold value. Hence, in an embodiment, a higher threshold value is used for younger patients as compared to older patients.

As an illustrative, but currently preferred, embodiment, the defined age is 67 years. This means that a first threshold value is used in the comparison with the determined level of STK1 material if the patient is 67 years old or older, whereas a second, preferably higher, threshold value is used in the comparison if the patient is younger than 67 years.

As is shown in Table 5, selecting a threshold value within the range of 0.35 µg/L to 0.60 µg/L, and in particular 0.45 µg/L enabled a differentiation of patients having a high hazard ratio for relapse and thereby high risk for cancer relapse versus patients having a low hazard ratio for relapse for patients 67 years old or older. In fact, using a higher threshold value actually lowered the p-value for differentiating high versus low hazard ratios. This is in clear contrast to Table 6, showing the corresponding hazard ratio for relapse for patients younger than 67 years old. For such younger patients higher threshold values, at least up to 1.05 µg/L, were associated with lower p-values for differentiating high versus low hazard ratios. Hence, for the older patient group (age ≥ 67 years), the optimal threshold value is within the range of 0.35 µg/L to 0.60 µg/L, and in particular 0.45 µg/L, when predicting cancer relapse, whereas for the younger patient group (age < 67 years), the optimal threshold value is above 0.75 µg/L but below 2.5 µg/L, such as about 1 µg/L.

Experimental data as present herein indicated that even an approximately twofold increase in STK1 levels for older patients was correlated with a large increase in the hazard ratios. Furthermore, the hazard ratios were only moderately further increased at higher STK1 levels for older patients. This means that in this elderly patient group, higher STK1 levels are not proportionally related to higher risk for cancer relapse in patients at higher age. This finding put demands to the STK1 measurement methods and in particular the sensitivity and robustness of the STK1 measurement methods to be able to detect also comparatively low STK1 levels in a biological sample and differentiate such low STK1 levels from corresponding STK1 levels in healthy subjects.

The antibody or the fragment thereof specifically binds to the STK1 material, and in particular binds specifically to the serum form of the TK1 protein.

The specificity of an antibody or a fragment thereof can be determined based on affinity and/or avidity. The affinity, represented by the equilibrium constant for the dissociation of an antigen with the antibody or the fragment thereof (K_{d}), is a measure for the binding strength between an antigenic determinant and an antigen-binding site on the antibody or the fragment thereof. The lesser the value of K_{d}, the stronger the binding strength between the antigenic determinant and the antibody or the fragment thereof. Alternatively, the affinity can also be expressed as the affinity constant (Kₐ), which is 1/K_{d}. As will be clear to the skilled person, affinity can be determined in a manner known per se, depending on the specific antigen of interest.

Avidity is the measure of the strength of binding between an antibody or a fragment thereof and the pertinent antigen. Avidity is related to both the affinity between an antigenic determinant and its antigen binding site on the antibody or the fragment thereof and the number of pertinent binding sites present on the antibody or the fragment thereof.

Typically, antibodies will bind to their antigen with a dissociation constant (K_{d}) of 10⁵ to 10⁻¹² moles/liter (M) or less, and preferably 10⁻⁷ to 10⁻¹² M or less and more preferably 10⁸ to 10⁻¹² M, i.e. with an association constant (Kₐ) of 10⁵ to 10¹² M⁻¹ or more, and preferably 10⁷ to 10¹² M⁻¹ or more and more preferably 10⁸ to 10¹² M⁻¹.

Generally, any K_{d} value greater than 10⁻⁴ M (or any Kₐ value lower than 10⁴ M⁻¹) is generally considered to indicate non-specific binding. Preferably, an antibody or a fragment thereof will bind to the STK1 material with an affinity less than 500 nM, preferably less than 200 nM, more preferably less than 10 nM, such as less than 5 nM or even lower, such as 1 nM or lower.

Specific binding of an antibody or a fragment thereof to an antigen or antigenic determinant can be determined in any suitable manner known per se, including, for example, Scatchard analysis and/or competitive binding assays, such as radioimmunoassays (RIA), enzyme immunoassays (EIA) and sandwich competition assays, and the different variants thereof known per se in the art.

In an embodiment, the antibody is a monoclonal antibody, i.e., a monoclonal anti-TK1 antibody. In another embodiment, the antibody is a polyclonal antibody, i.e., a polyclonal anti-TK1 antibody.

In an embodiment, the antibody or the fragment thereof has specificity for an epitope or peptide consisting of an amino acid sequence from the C-terminal region of TK1, preferably of human TK1.

The peptide is preferably selected from a portion of TK1 ranging from amino acid position 200 to the end of the TK1, i.e., amino acid position 234 in humans (SEQ ID NO: 28). In a particular embodiment, the peptide is selected from a portion of the TK1 protein ranging from amino acid position 205, preferably 210 to amino acid position 230, preferably 225.

The peptide is preferably an N-mer, wherein N is an integer within a range of from 8 up to 20, preferably within a range of from 10 up to 15. The peptide preferably consists of N consecutive amino acids in the C-terminal region of the TK1 protein.

In an embodiment, the peptide consists of the following amino acid sequence GEAVAARKLF (SEQ ID NO: 1). In another embodiment, the peptide consists of the following amino acid sequence NCPVPGKPGE (SEQ ID NO: 2). In a further embodiment, the peptide consists of the following amino acid sequence PVPGKPGEAV (SEQ ID NO: 3). In yet another embodiment, the peptide consists of the following amino acid sequence NCPVPGKPGEAV (SEQ ID NO: 4).

A monoclonal antibody having specificity for an epitope consisting of GEAVAARKLF (SEQ ID NO: 1) has a variable heavy (VH) domain complementarity determining region 1 (CDR1) having amino acid sequence DYEMH (SEQ ID NO: 5), a VH domain CDR2 having amino acid sequence AIHPGYGGTAYNQKFKG (SEQ ID NO: 6), a VH domain CDR3 having amino acid sequence FITKFDY (SEQ ID NO: 7), a variable light (VL) domain CDR1 having amino acid sequence KSSQSLLDSDGKTFLN (SEQ ID NO: 8), a VL domain CDR2 having amino acid sequence LVSKLDS (SEQ ID NO: 9) and a VL domain CDR3 having amino acid sequence WQGTHFPWT (SEQ ID NO: 10).

A monoclonal antibody having specificity for the epitopes NCPVPGKPGE (SEQ ID NO: 2), PVPGKPGEAV (SEQ ID NO: 3) and NCPVPGKPGEAV (SEQ ID NO: 4) has a VH domain CDR1 having amino acid sequence DYEMH (SEQ ID NO: 5), a VH domain CDR2 having amino acid sequence AILPGSGGTAYNQKFKG (SEQ ID NO: 11), a VH domain CDR3 having amino acid sequence LITTFDY (SEQ ID NO: 12), a VL domain CDR1 having amino acid sequence KSSQSLLDSDGKTYLN (SEQ ID NO: 13), a VL domain CDR2 having amino acid sequence LVSKLDS (SEQ ID NO: 9), and a VL domain CDR3 having amino acid sequence WQGTHFPWT (SEQ ID NO: 10).

In another embodiment, the antibody or the fragment thereof has specificity for a conformation dependent epitope of human TK1. A monoclonal antibody having specificity for such a conformation dependent epitope has a VH domain CDR1 having amino acid sequence SGYSWH (SEQ ID NO: 14), a VH domain CDR2 having amino acid sequence YIHYSGSTTYNPSLKG (SEQ ID NO: 15), a VH domain CDR3 having amino acid sequence WGTGHWYFDV (SEQ ID NO: 16), a VL domain CDR1 having amino acid sequence RSSTGAVTTTNYAN (SEQ ID NO: 17), a VL domain CDR2 having amino acid sequence GTNNRVP (SEQ ID NO: 18), and a VL domain CDR3 having amino acid sequence ALWYSNHWV (SEQ ID NO: 19).

The above-three presented examples of monoclonal anti-TK1 antibodies that can be used according to the embodiments are further disclosed in WO 2015/094106, the teaching of which regarding monoclonal anti-TK1 antibodies is incorporated herein by reference.

Hence, in an embodiment, the monoclonal antibody or the fragment thereof is selected from the group consisting of a monoclonal antibody or a fragment thereof having specificity for GEAVAARKLF (SEQ ID NO: 1) of human TK1, a monoclonal antibody or a fragment thereof having specificity for at least one of NCPVPGKPGE (SEQ ID NO: 2), PVPGKPGEAV (SEQ ID NO: 3) and NCPVPGKPGEAV (SEQ ID NO: 4) of human TK1, and a monoclonal antibody or a fragment thereof having specificity for a conformation dependent epitope of human TK1.

In another embodiment, the antibody or the fragment thereof has specificity for an epitope or peptide consisting of KPGEAVAARKLFAPQ (SEQ ID NO: 20). At least one additional amino acid, such as a cysteine residue, may be added to the N-terminal or C-terminal, preferably the N-terminal, of the peptide for use as coupling to other molecules, such as carrier proteins.

An antibody having specificity for this epitope is further disclosed in WO 95/29192, the teaching of which regarding anti-TK1 antibodies is incorporated herein by reference.

In a further embodiment, the antibody or the fragment thereof has specificity for an epitope or peptide consisting of an amino acid sequence from an active site of TK1. The peptide is preferably selected from a portion of TK1 ranging from amino acid position 150 to amino acid position 190 in human TK1. In a particular embodiment, the peptide is selected from a portion of TK1 ranging from amino acid position 155, preferably 160 and more preferably 161, to amino acid position 185, preferably 183.

The peptide is preferably an M-mer, wherein M is an integer within a range of from 10 up to 40, preferably within a range from 20 up to 30 and more preferably 23 or 24. The peptide preferably consists of M consecutive amino acids in the active site of the TK1 protein.

At least one additional amino acid, such as a cysteine residue, may be added to the N-terminal or C-terminal, preferably the N-terminal, of the peptide for use as coupling to other molecules, such as carrier proteins.

In an embodiment, the peptide consisting of an amino acid sequence from the active site of TK1 has an amino acid sequence corresponding to amino acid positions 161 to 183 in human TK1, i.e., has amino acid sequence of AYTKRLGTEKEVEVIGGADKYHS (SEQ ID NO: 21).

An antibody having specificity for this epitope is further disclosed in WO 2008/142664, the teaching of which regarding anti-TK1 antibodies is incorporated herein by reference.

In a further embodiment, the antibody or the fragment thereof is a monoclonal antibody or a fragment thereof as disclosed in WO 2019/201901, the teaching of which regarding monoclonal anti-TK1 antibodies is incorporated herein by reference.

For instance, the monoclonal antibody could be mAb 6C6, mAb 4H4 or mAb 23C11.
mAb 6C6 VH domain (SEQ ID NO: 22): 
METGLRWLLLVAVLKGVQCQEQLEESGGDLVKPEGSLTLTCTASRFSFSSSYWICWVRQAPGKGLEW
IACIYAGDSGSSYYASWAKGRFTVSKTSSTTVTLQTTSLTAADTATYFCARASVGAAYDYFALWGPGTL
VTVSSGQPKAPSVFPLAPCCGDTPSSTVTLGCLVKGYLPEPVTVTWNSG
mAb 6C6 VL domain (SEQ ID NO: 23): 
MDTRAPTQLLGLLLLWLPGARCALVMTQTPASVEAAMGGTVTIKCQASEDVSSHLAWYQQRPGQPPK
LLIYGASDLASGVPSRFTGSGSGTQFTLAISDLECADAATYYCQGYYYISDSPYVFGGGTEVVVKGDPV
APTVLIFPPAADQVATGTVTIVCVANKYFPDVTVTWEVDGTTQTTGIENSKTPQNSADCTYNLSSTLTLT
STQYNSHKEYTCKVTQGTTSVVQSFNRGDC
mAb 4H4 VH domain (SEQ ID NO: 24): 
METGLRWLLLVAVLKGVQCQSLEESGGGLVQPEGSLTLTCTASGFSFSSGYDMCWVRQTPGKGLEWI
ACISVDSDGVTYYASWAKGRFTISKTSSTTVTLQMTSLTAADTATYFCARGYESSSGVYIPYFTLWGPG
TLVTVSSGQPKAPSVFPLAPCCGDTPSSTVTLGCLVKGYLPEPVTVTWNSG
mAb 4H4 VL domain (SEQ ID NO: 25): 
MDMRAPTQLLGLLLLWLPGARCADIVLTQTPASVEAAVGGTVTIKCQASQSIYSYLAWYQHKPGQPPK
LLIYKASTLASGVPSRFKGSGSGTEYTLTISDLECADAATYYCQHYYYSSTSGGGVFGGGTEVVVKGD
PVAPTVLIFPPAADQVATGTVTIVCVANKYFPDVTVTWEVDGTTQTTGIENSKTPQNSADCTYNLSSTLT
LTSTQYNSHKEYTCKVTQGTTSVVQSFNRGDC
mAb 23C11 VH domain (SEQ ID NO: 26): 
METGLRWLLLVAVLKGVQCQSLEESGGRLVTPGTPLTLTCTASGFSLSNYYMSWVRQAPGKGLEWIGI
IYGDDNTYCANWTKGRFTISKTSTTVDLTITSPTTEDTATYFCARGPDYIAAKMDIWGPGTLVTVSLGQP
KAPSVFPLAPCCGDTPSSTVTLGCLVKGYLPEPVTVTWNSG
mAv 23C11 VL domain (SEQ ID NO: 27):

MDTRAPTQLLGLLLLWLPGARCDVVMTQTPASVEAAVGGTVTIKCQASQSISGYLSWYQQKPGQRPK
LLIYRASTLESGVPSRFKGSGSGTEFTLTISDLECADAATYYCQCTYGSSTFSSYGNAFGGGTEVVVKG
DPVAPTVLIFPPAADQVATGTVTIVCVANKYFPDVTVTWEVDGTTQTTGIENSKTPQNSADCTYNLSST
LTLTSTQYNSHKEYTCKVTQGTTSVVQSFNRGDC

In an embodiment, the level of STK1 material in the body sample is determined using a kit. The kit preferably comprises a first antibody or a first fragment thereof and a second antibody or a second fragment thereof. The first and second antibodies can be selected from the above described illustrative examples of monoclonal and polyclonal anti-TK1 antibodies.

In a particular embodiment, the kit comprises a first monoclonal antibody or a first fragment thereof having specificity for an epitope selected from the group consisting of i) GEAVAARKLF (SEQ ID NO: 1) of human TK1, ii) at least one of NCPVPGKPGE (SEQ ID NO: 2), PVPGKPGEAV (SEQ ID NO: 3) and NCPVPGKPGEAV (SEQ ID NO: 4) of human TK1, and iii) a conformation dependent epitope of human TK1. The kit also comprises a second monoclonal antibody or a second fragment thereof having specificity for an epitope selected from the group consisting of i) GEAVAARKLF (SEQ ID NO: 1) of human TK1, ii) at least one of NCPVPGKPGE (SEQ ID NO: 2), PVPGKPGEAV (SEQ ID NO: 3) and NCPVPGKPGEAV (SEQ ID NO: 4) of human TK1, and iii) a conformation dependent epitope of human TK1.

In an embodiment, the first antibody or the first fragment thereof is a so-called capture antibody immobilized to a support or intended to be immobilized to the support and the second antibody or the second fragment thereof is a so-called detection antibody. In another embodiment, the second antibody or the second fragment thereof is the capture antibody immobilized to the support or intended to be immobilized to the support, whereas the first antibody or the first fragment thereof is used as detection antibody.

In an embodiment, the first and second antibodies or the first and second fragments have specificities for different epitopes in the STK1 material.

In another embodiment, the first and second antibodies or the first and second fragments have specificities for the same epitope in the STK1 material. This is possible since the same epitope may be present at multiple copies in the high molecular weight complexes of multiple TK1 protein units. Thus, the STK1 material may be a multivalent complex of multiple, i.e., at least two, TK1 protein units. In fact, the same type of antibody or fragment thereof could be used as the first and second antibody or first and second fragment.

In an embodiment, one of the first and the second antibody or the first and second fragment has specificity for a peptide consisting of an amino acid sequence from the active site of TK1 and the other of the first and second antibody or the first and second fragment has specificity for a peptide consisting of an amino acid sequence from the C-terminal region of TK1.

In another embodiment, one of the first and the second antibody or the first and second fragment has specificity for a peptide consisting of a first amino acid sequence from the C-terminal region of TK1 and the other of the first and the second antibody or the first and second fragment has specificity for a peptide consisting of the first amino acid sequence from the C-terminal region of TK1 or a second, different amino acid sequence from the C-terminal region of TK1.

In a further embodiment, one of the first and the second antibody or the first and second fragment has specificity for a peptide consisting of a first amino acid sequence from the C-terminal region of TK1 and the other of the first and the second antibody or the first and second fragment has specificity for a conformation dependent epitope of human TK1.

In yet another embodiment, one of the first and the second antibody or the first and second fragment has specificity for a peptide consisting of an amino acid sequence from the active site of TK1 and the other of the first and second antibody or the first and second fragment has specificity for a conformation dependent epitope of human TK1.

A fragment of an antibody as used herein can be selected from a group consisting of a single chain antibody, a Fv fragment, a scFv fragment, a Fab fragment, a F(ab')₂ fragment, a Fab' fragment, a Fd fragment, a single-domain antibody (sdAb), a scFv-Fc fragment, a di-scFv fragment and a CDR region.

In an embodiment, the kit is a sandwich assay kit. In a particular embodiment, the kit is an Enzyme-Linked Immunosorbent Assay (ELISA) kit and preferably a sandwich ELISA.

In the discussion below, the first antibody or first fragment is assumed to be the capture antibody with the second antibody or second fragment acting as detection antibody. The embodiments are, however, not limited thereto but could switch capture and detection antibodies.

A sandwich ELISA can be used to detect STK1 material in a body sample by preparing a surface of a support, such as a solid support, to which the first antibody or the first fragment is bound as so-called capture antibody. In a preferred embodiment, a known quantity of the first antibody or the first fragment is bound to the surface of the support. Any non-specific binding sites on the surface are optionally, but preferably, blocked. The body sample is then applied to the surface so that any STK1 material present therein will be captured by the immobilized first antibodies or first fragments. Unbound material is preferably removed by one or multiple washing steps. The second antibody or second fragment, typically denoted detection antibody, is then added and is allowed to bind to any STK1 material captured by the first antibody or the first fragment.

The amount of bound second antibody or second fragment is then determined by direct or indirect detection methods. For instance, a label or enzyme can be attached directly to the second antibody or the second fragment or indirectly via a link, such as a biotin-streptavidin or a biotin-avidin link. It is, alternatively, possible to use a secondary antibody or second fragment that is labeled or connected to an enzyme and binds specifically to the second antibody or second fragment.

Hence, in an embodiment the second antibody or second fragment has a covalently attached biotin. Alternatively, the second antibody or second fragment has a covalently attached streptavidin or avidin.

The kit preferably also comprises a horseradish peroxidase (HRP) labeled streptavidin or a HRP labeled avidin. Alternatively, the kit also comprises a HRP labeled biotin. The kit also comprises a HRP substrate, such as a 3,3',5,5'-tetramethylbenzidine (TMB) substrate, a 3,3'-diaminobenzidine (DAB) substrate or a 2,2'-azino-bis(3-ethylbenzothiazoline-6-sulphonic acid) (ABTS) substrate. In such a case, the level of STK1 material in the sample can be determined by spectrophotometric methods that detect the conversion of the chromogenic substrate by HRP into a colored product that is detectable.

In an embodiment, the kit also comprises a microtiter plate (MCP) as the support to which the first antibody or first fragment is immobilized or is intended to be immobilized.

In an embodiment, one of the first antibody or the first fragment and the second antibody or the second fragment has specificity for an epitope consisting of GEAVAARKLF (SEQ ID NO: 1) and has a VH domain CDR1 having amino acid sequence DYEMH (SEQ ID NO: 5), a VH domain CDR2 having amino acid sequence AIHPGYGGTAYNQKFKG (SEQ ID NO: 6), a VH domain CDR3 having amino acid sequence FITKFDY (SEQ ID NO: 7), a VL domain CDR1 having amino acid sequence KSSQSLLDSDGKTFLN (SEQ ID NO: 8), a VL domain CDR2 having amino acid sequence LVSKLDS (SEQ ID NO: 9) and a VL domain CDR3 having amino acid sequence WQGTHFPWT (SEQ ID NO: 10).

In a particular embodiment, the other of the first antibody or the first fragment and the second antibody or the second fragment has specificity for the epitopes NCPVPGKPGE (SEQ ID NO: 2), PVPGKPGEAV (SEQ ID NO: 3) and NCPVPGKPGEAV (SEQ ID NO: 4) and has a VH domain CDR1 having amino acid sequence DYEMH (SEQ ID NO: 5), a VH domain CDR2 having amino acid sequence AILPGSGGTAYNQKFKG (SEQ ID NO: 11), a VH domain CDR3 having amino acid sequence LITTFDY (SEQ ID NO: 12), a VL domain CDR1 having amino acid sequence KSSQSLLDSDGKTYLN (SEQ ID NO: 13), a VL domain CDR2 having amino acid sequence LVSKLDS (SEQ ID NO: 9), and a VL domain CDR3 having amino acid sequence WQGTHFPWT (SEQ ID NO: 10).

The kit does not necessarily have to be an ELISA kit. In another embodiment, the kit uses affinity chromatography where the first antibody is bound to the stationary phase, such as to a gel matrix or beads in a column. For instance, the gel matrix or beads could be made of agarose, such as SEPHAROSE^{®}.

In such a case, TK1 material present in a body sample will be entrapped in the column through binding to the immobilized first antibodies or first fragments. Following washing, the bound STK1 material can be eluted and detected using the second antibody or second fragment. For instance, the amount of eluted STK1 material can be determined using Western blotting and with the second antibody or second fragment for STK1 detection using direct or indirect detection methods.

The support could alternatively be magnetic beads, such as DYNABEADS^{®} magnetic beads.

In a further embodiment, the kit is a chemiluminescence immunoassay (CLIA) kit. CLIA is an immunoassay technique where the label is a luminescent molecule. CLIA methods can be direct, using luminophore markers, or indirect, using enzyme markers. Either method may be competitive or non-competitive. In direct CLIA methods, the luminophore markers used are typically acridinium and ruthenium esters, while the enzymatic markers used in indirect methods are typically alkaline phosphatase with adamantyl 1,2-dioxetane aryl phosphate (AMPPD) substrate and HRP with luminol or its derivatives as substrate.

The kit does not necessarily have to include two antibodies or fragment but could instead include only one type of antibodies or fragments.

Furthermore, the kit does not necessarily have to comprise a so-called capture antibody or fragment. In clear contrast, multiple, i.e., at least two, different antibodies or fragments could be used to determine the level of STK1 material without the need for immobilizing at least one of the antibodies or fragments.

In an embodiment, the method also comprises selecting an anti-cancer treatment for the patient based on the predicted risk of cancer relapse. Thus, an optimal or at least suitable anti-cancer treatment is selected for hematological cancer patient based on the determined level of STK1 material in the body sample and thereby based on the predicted cancer relapse risk estimated for the patient based on the comparison between determined level of STK1 material in the body sample and the selected threshold value. This means that patients with a determined level of STK1 material higher than the selected threshold value and thereby a predicted high risk of cancer relapse could be selected for a more aggressive anti-cancer treatment as compared to patients with a level of STK1 material below the threshold value and thereby a predicted lower risk of cancer relapse. Examples of anti-cancer treatments that can be selected include one or more of symptomatic treatment, e.g., blood transfusions, to chemotherapy, radiotherapy, immunotherapy and bone marrow transplant.

For instance, patients having a determined level of STK1 material exceeding the previously mentioned selected threshold value could be selected for a first anti-cancer treatment, whereas other patients with a determined level of STK1 material below the selected threshold value are selected for a second, different anti-cancer treatment.

In an embodiment, the method comprises selecting a patient surveillance schedule for the patient based on the predicted cancer relapse risk of the patient. Thus, an optimal or at least suitable patient surveillance schedule or scheme is selected for the patient based on the comparison between determined level of STK1 material in the body sample and the selected threshold value and thereby based on the predicted cancer relapse risk estimated for the patient based on the determined level of STK1 material in the body sample. This means that patients with a determined level of STK1 material above the selected threshold value and thereby a predicted high risk for cancer relapse could be selected for a more frequent surveillance and follow-up (first surveillance schedule) as compared to patients with a level of STK1 material below the selected threshold value and thereby a predicted lower risk of cancer relapse, which instead can follow a less frequent surveillance and follow-up (second surveillance schedule).

### EXAMPLES

Serum levels of thymidine kinase 1 (TK1) from 146 patients (64 women, 82 men) suffering from diffuse large B-cell lymphoma (DLBCL) and treated with R-CHOP were measured by an immunoassay, before, during and after treatment. TK1 concentrations in serum were found significantly raised in patients with lymphoma, compared to TK1 concentrations in healthy individuals (p<0.00001). Higher concentrations correlated with more advanced stage and high International Prognostic Index (IPI) (p<0.00001). Elevated TK1 before treatment correlated with shorter survival (p<0.001). Overall, TK1 concentration increased about ten-fold during treatment and declined afterwards. The changes were most prominent in patients with stage 1 and 2. In conclusion, TK1 measured with an immunoassay can be correlated to DLBCL characteristics and outcome and pose an important reference value in treatment evaluation.

### Materials and Methods

### Study design and settings

Serum and clinical data for 146 patients with diagnosed with DLBCL between year 2010 and 2016 were obtained from the Uppsala-Umeå Comprehensive Cancer Consortium (U-CAN). The core of the U-CAN project is to establish a longitudinal collection of data, blood and tissue samples together with informed consent from cancer patients for future research. Within the framework of U-CAN, blood samples are to be obtained for biobanking at several pre-defined time points during the course of cancer diagnosis and subsequent treatment. For patients with DLBCL these time points are defined as; at diagnosis (or at least before start of treatment), in the middle of the treatment period (i.e., after 3 treatment cycles), 3 months and 1 year after treatment end, and at relapse. The blood sample during treatment was not introduced at U-CAN launch but was added to the protocol more recently. All patients included in this study were treated with the standard chemotherapy regimen R-CHOP (rituximab, cyclophosphamide, doxorubicin, vincristine, and prednisone), or equivalent, with curative intent and administered at 14- or 21-days intervals.

Baseline data were obtained via U-CAN and consisted of age at diagnosis; gender; Ann Arbor stage; B-symptoms (fever >38 °C, night sweat, weight loss >10% last 6 months); bulky disease (defined as tumor with a transverse diameter of > 7.5 cm); performance status according to Eastern Cooperative Oncology Group (ECOG-PS); International prognostic index (IPI) score; age adjusted IPI (aaIPI); type of treatment; treatment outcome, which was defined according to International response criteria (Cheson et al., Revised Response Criteria for Malignant Lymphoma. J Clin Oncol. 2007; 25:579-586).

TK1 was measured in serum by an immunoassay (AroCell TK 210 ELISA) provided by AroCell AB, Uppsala, Sweden. The assay was run according to the instructions of the manufacturer. The imprecision of the assay was less than 10% standard deviation (CV) as judged by measuring duplicate samples. The lower limit of quantitation, i.e., when CV% exceeded 20% of duplicates, was 0.20 µg/L (Kumar et al., AroCell TK 210 ELISA for determination of TK1 protein: age-related reference ranges and comparison with other TK1 assays. BioTechniques. 2020; btn-2019-0148.41).

Non-infected and apparently healthy persons served as controls. Fifty-seven males (median age 42 years, interquartile (IQ) range 29.7-52.2 years) and 88 females (median 45.5, IQ range 34.5-57.5 years) were included.

### Statistics

Disease free survival (DFS) was calculated from date of diagnosis until documented relapse or last follow-up. Patients never achieving complete remission were considered a DFS of zero days. Disease specific survival (DSS) was calculated from the date of diagnosis until date of death with remaining lymphoma or last follow-up. Overall survival (OS) was calculated from date of diagnosis until death or last follow up.

Non-parametric statistics was used throughout the Example unless otherwise indicated. The Mann-Whitney U test was used for the comparison of two groups and the Kruskal-Wallis analysis of variance for the comparison of more than two groups. The Wilcoxon's test was used to compare paired samples. The p-values are indicted in the text or on figures and p<0.05 was considered significant. Correlations between variables were calculated by Spearman rank. Estimates of DFS, DSS and OS were calculated by Kaplan-Meier statistics and associated log-rank test. Cox proportional hazard test was used for multivariate time dependent analyses. The statistical program Medcalc (MedCalc Statistical Software version 19.2.6 (MedCalc Software Ltd, Ostend, Belgium; https://www.medcalc.org; 2020) and the statistical programme R version 3.4.3 (R Foundation for Statistical Computing; https://www.R-project.org/) were used for all analyses.

### Ethics

This study was approved by the local Ethics Board in Uppsala, Sweden (Dnr 2014/233). The study was also approved by the U-CAN study selection committee. Written informed consent had, thus, previously been obtained within the U-CAN program.

### Results

### Patients and treatment

Patient characteristics are summarized in Table 1. There were 44 patients who died during the follow up period whereas 31 died with remaining lymphoma.

**Table 1 - Patient Baseline characteristics of 146 patients with DLBCL**

| | **All** | **High initial TK1 (>0.45 µg/L)** | **Normal initial TK1 (<0.45 µg/L)** | **p-value** |
|---|---|---|---|---|
| n | 146 | 67 | 72 | |
| Age (years) | 63.8 (18-90) | 63.2 (18-85) | 64.4 (26-90) | 0.661 |
| Follow up time (months) | 50.7 (1-100.6) | 43.9 (1-98.3) | 58.3 (8.1-100.6) | |
| Deaths | 44 | 25 | 19 | |
| Deaths with remaining lymphoma | 31 | 22 | 9 | |

| Gender | | | | |
|---|---|---|---|---|
| Female | 64 | 28 | 32 | 0.885² |
| Male | 82 | 39 | 40 | |

| ECOG Performance status | | | | |
|---|---|---|---|---|
| 0 | 102 | 40 | 62 | 0.0053 |
| 1 | 19 | 14 | 5 | |
| 2 | 8 | 7 | 1 | |
| 3 | 4 | 2 | 2 | |
| NA | 13 | 4 | 2 | |

| Ann Arbor clinical stage | | | | |
|---|---|---|---|---|
| I | 39 | 5 | 34 | 0.0005³ |
| II | 21 | 9 | 12 | |
| III | 27 | 15 | 12 | |
| IV | 50 | 36 | 14 | |
| NA | 9 | 2 | 0 | |

| IPI | | | | |
|---|---|---|---|---|
| 0 | 18 | 7 | 11 | 0.0005³ |
| 1 | 36 | 7 | 29 | |
| 2 | 33 | 17 | 16 | |
| 3 | 31 | 22 | 9 | |
| 4 | 11 | 9 | 2 | |
| 5 | 1 | 0 | 1 | |
| NA | 16 | 5 | 4 | |

| aalPI | | | | |
|---|---|---|---|---|
| 0 | 41 | 9 | 32 | 0.0005³ |
| 1 | 45 | 20 | 25 | |
| 2 | 38 | 28 | 10 | |
| 3 | 7 | 6 | 1 | |
| NA | 15 | 4 | 4 | |

| Cell of origin | | | | |
|---|---|---|---|---|
| GC | 50 | 23 | 27 | 0.7² |
| Non-GC | 38 | 18 | 22 | |
| NA | 58 | 26 | 23 | |

| | | | | |
|---|---|---|---|---|
| ¹t-test two sided, ²χ², ³χ² with Monte Carlo simulation DLBCL: Diffuse Large B-cell Lymphoma; TK1: Thymidine Kinase 1; n: number; ECOG: Eastern Cooperative Oncology Group; NA: not available; IPI: International Prognostic Index; aalPl: age adjusted IPI; GC: Germinal centre | | | | |

### The relationship between TK1 in serum and disease

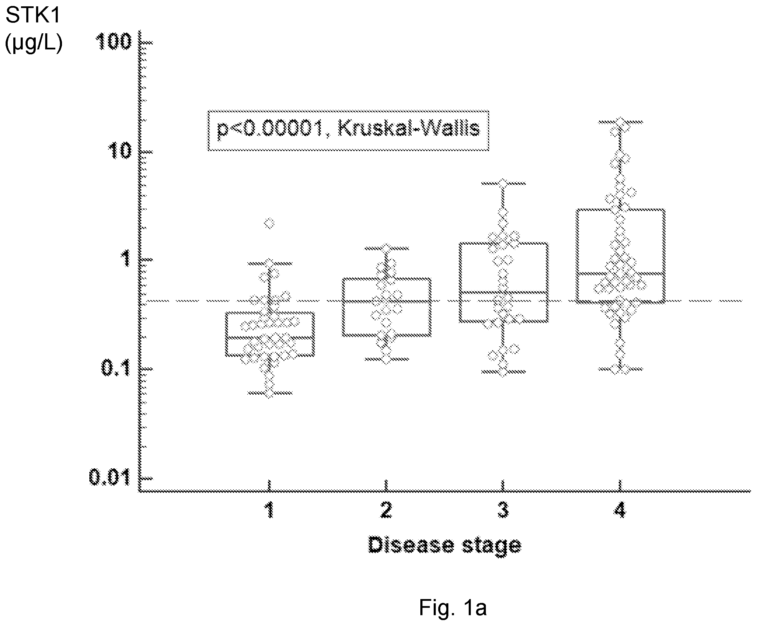

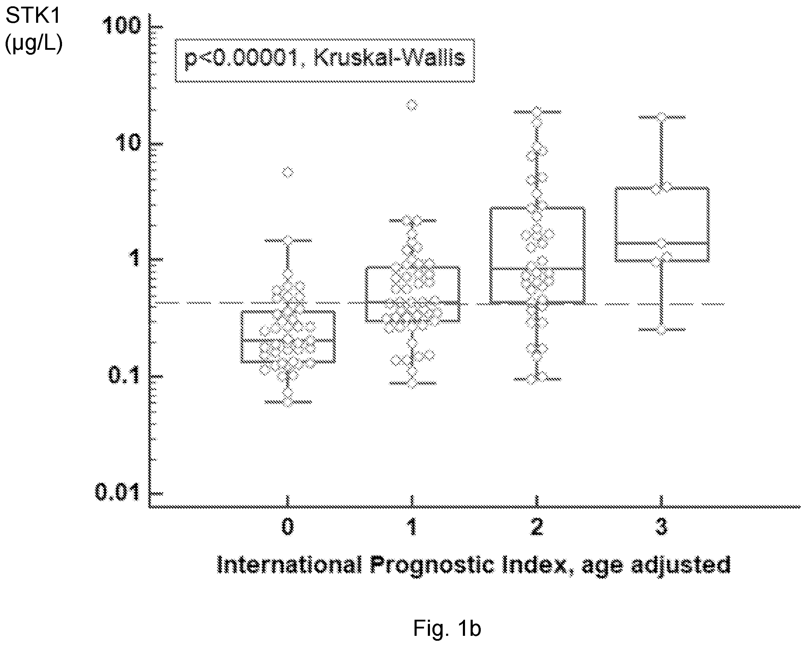

TK1 concentrations in serum, before treatment, were significantly raised in patients with lymphoma as compared to the concentrations in healthy individuals, median 0.44 µg/L (IQ range 0.22 to 1.06 µg/L) vs. median 0.22 µg/L (IQ range 0.18 to 0.29 µg/L), p<0.00001. Fig. 1a depicts the relationship between TK1 concentrations and disease stage, with increased TK1 concentrations at more advanced disease. The highest concentrations were found in patients with stage 4 (p<0.00001). In Fig. 1b a strong relationship to the International prognostic index (IPI) is also shown, p<0.00001.

### Relation of TK1 to outcome

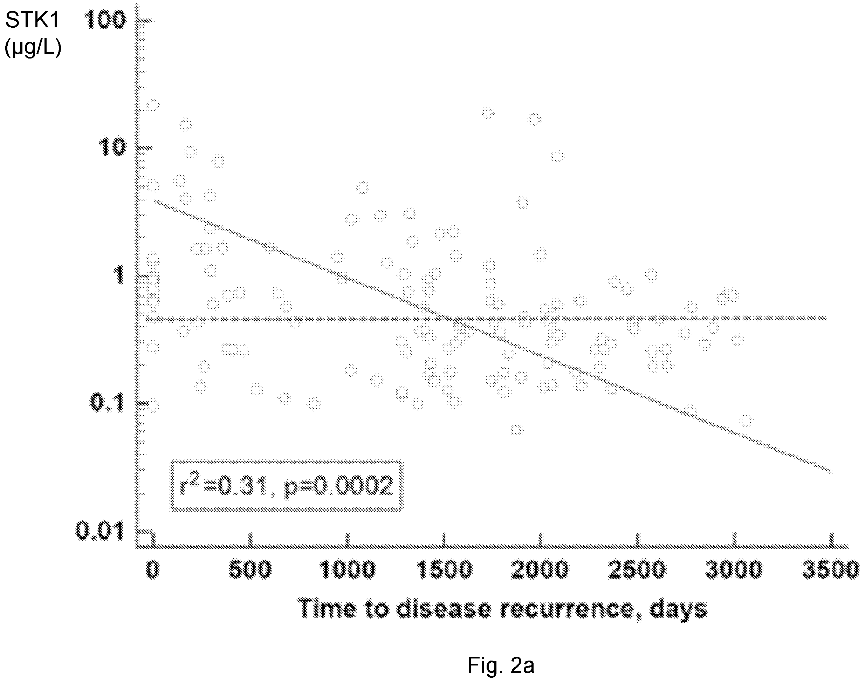

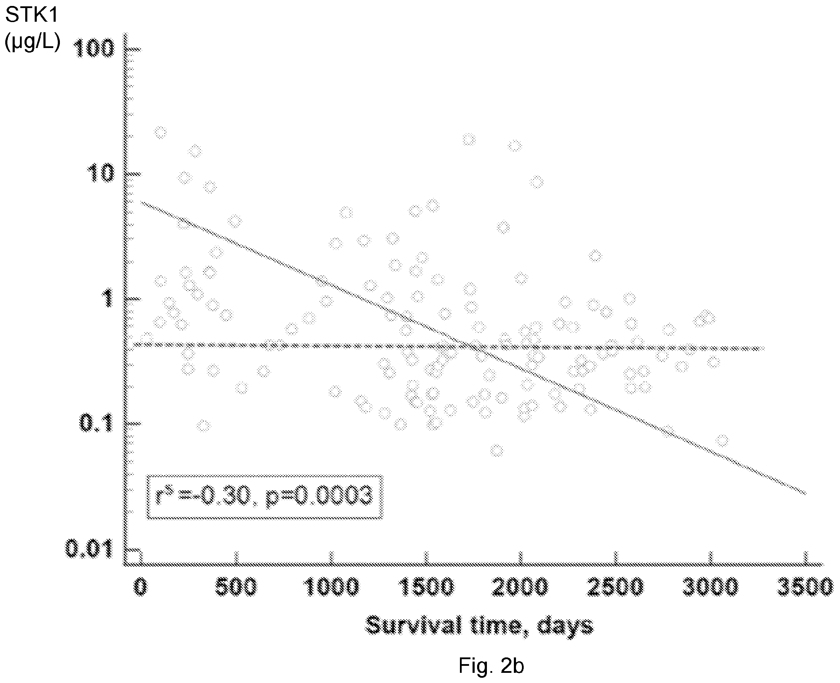

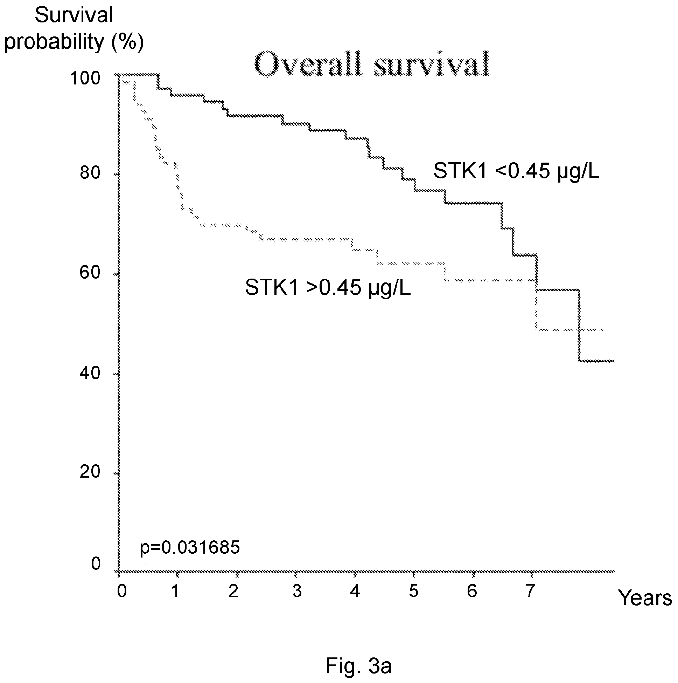

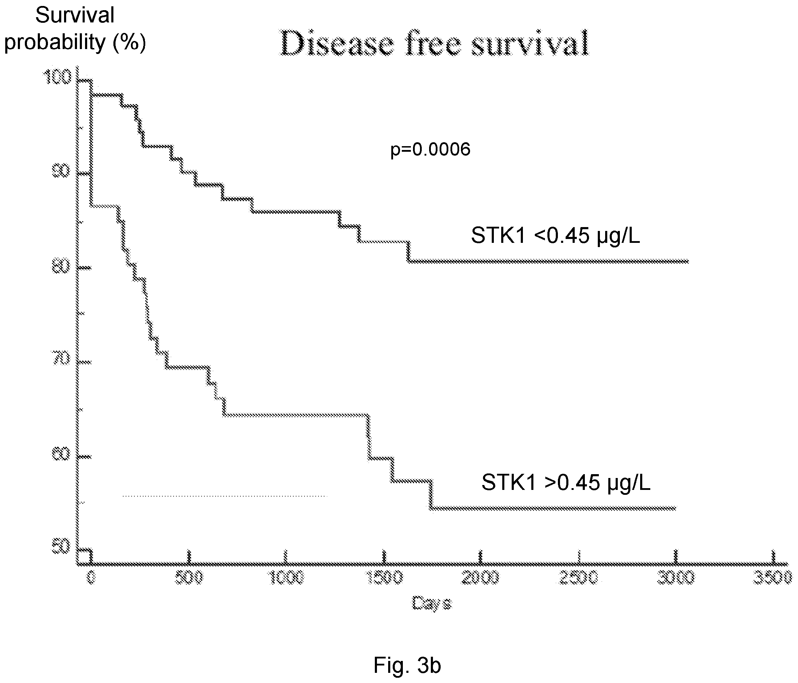

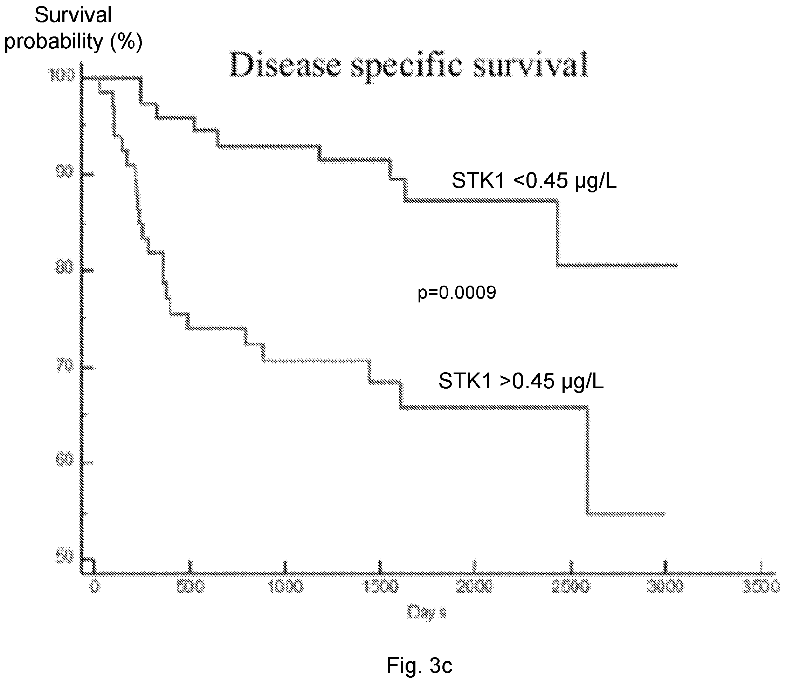

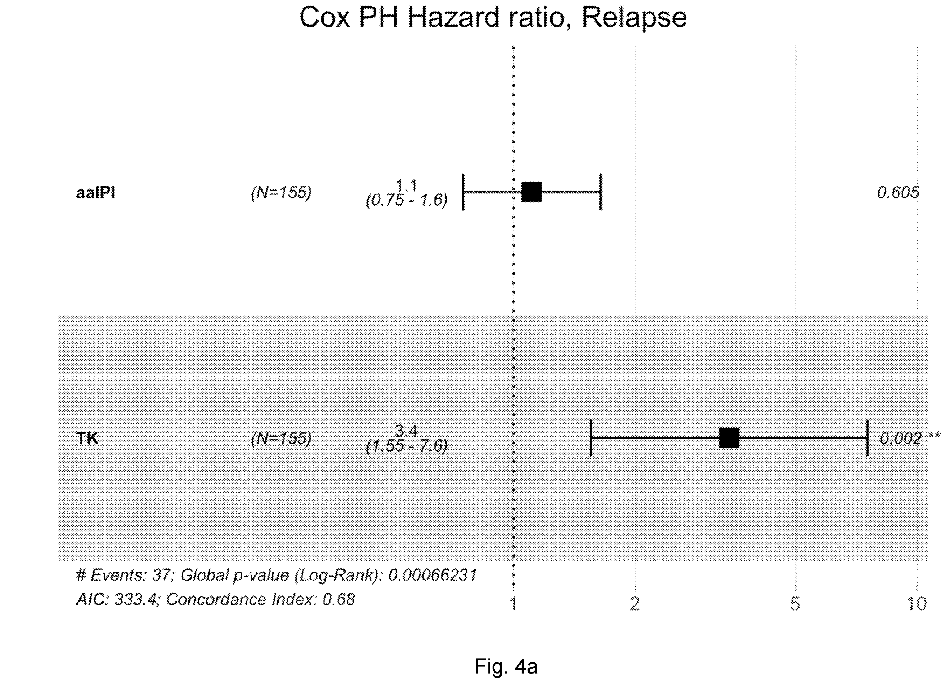

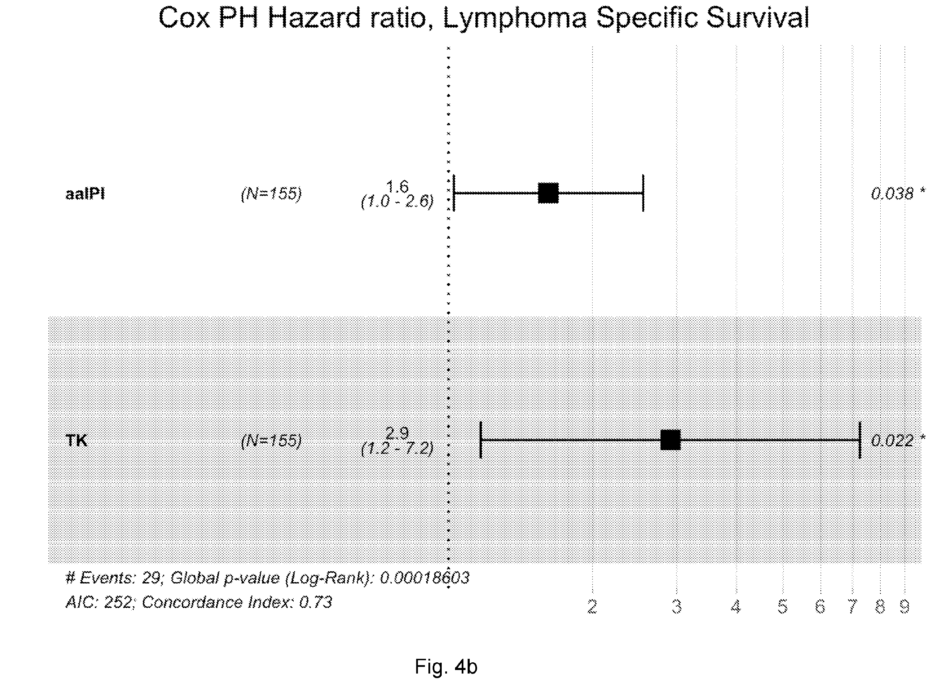

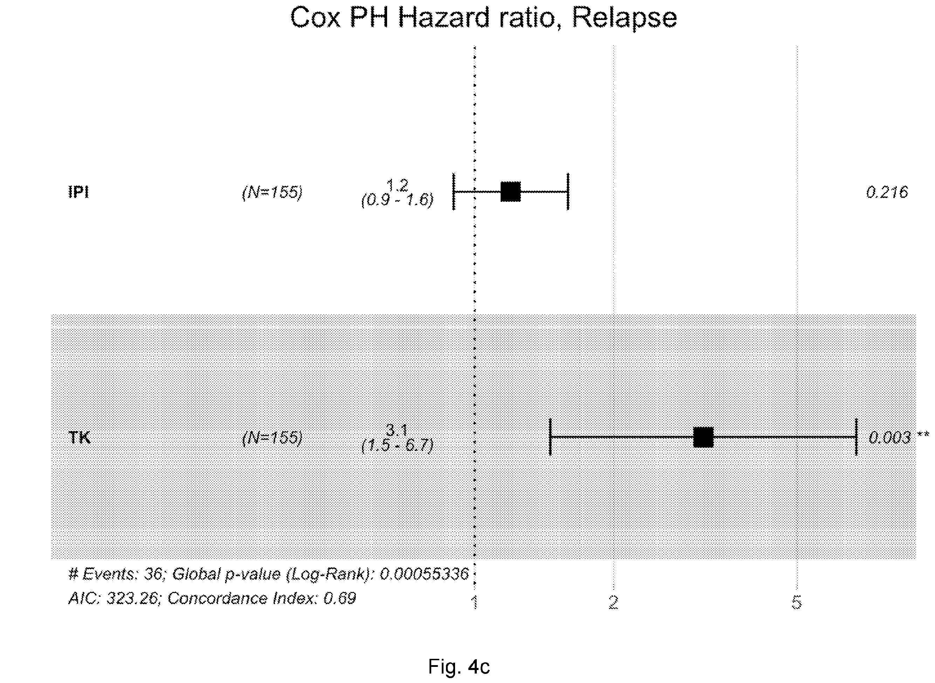

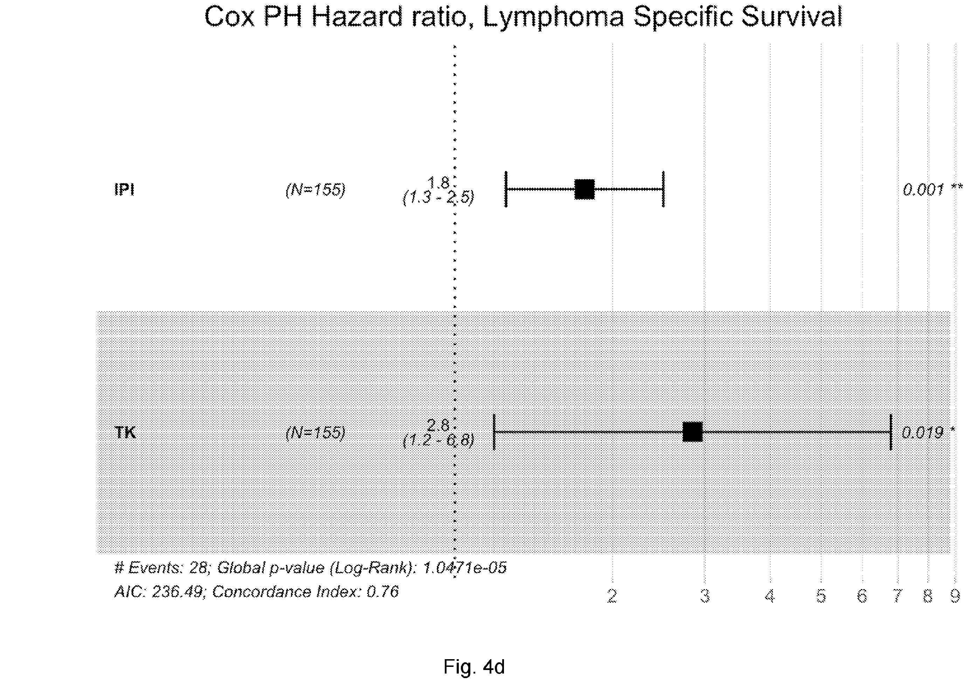

Time to disease relapse (Fig. 2a) and time to death (Fig. 2b) were both significantly correlated to serum concentrations of TK1 concentration in serum before treatment (rs=-0.30 and rs=-0.29, respectively, p<0.001 for both correlations), with the shortest time for the patients with the highest concentrations. By dichotomizing the TK1 concentrations before treatment by the upper limits for healthy individuals, i.e., 0.45 µg/L, the OS, DFS and DSS, as estimated by Kaplan-Meier and associated log-rank test (Figs. 3a to 3c), were most favorable for patients with normal concentrations (p=0.03, p=0.0006 and p=0.0009, respectively). In multivariate analyses, dichotomized TK1 was also found to be a significant prognostic factor together with each of aalPI and IPI, for DFS and DSS (p<0.01 and p<0.025, respectively) (Figs. 4a-4d). It is shown in Table 2 that patients with B-symptoms had significantly higher concentrations of TK1 in serum (p<0.0001).

**Table 2 - Median TK1 levels (µg/L) together with Q1 and Q3 range. Mann-Whitney U-test was applied.**

| | No | Yes | p |
|---|---|---|---|
| Bulky disease | 0.36 (0.18-0.76) | 0.55 (0.39-0.94) | 0.03 |
| B-symptom | 0.35 (0.18-0.64) | 0.95 (0.48-3.00) | <0.0001 |

### TK1 in relation to treatment

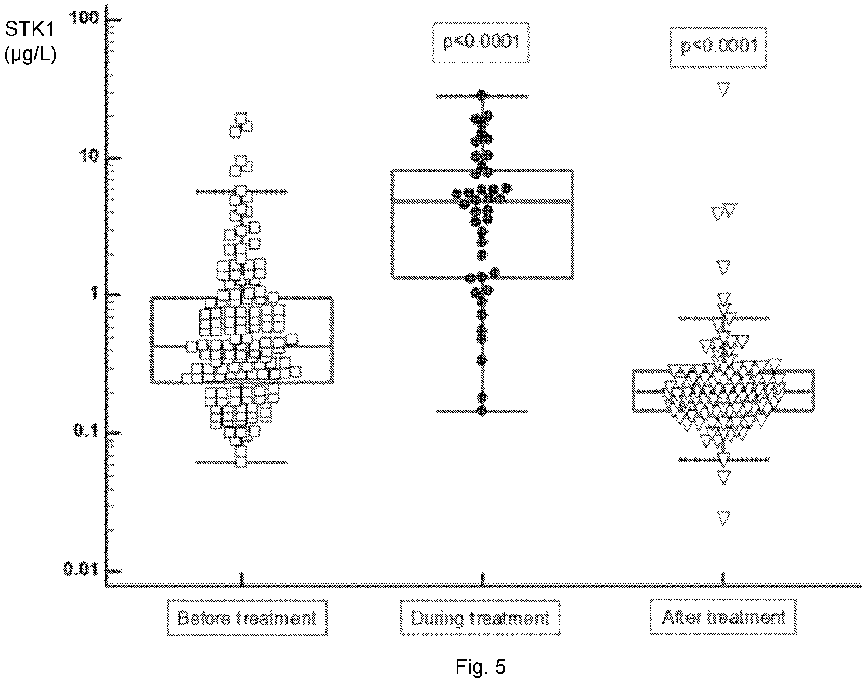

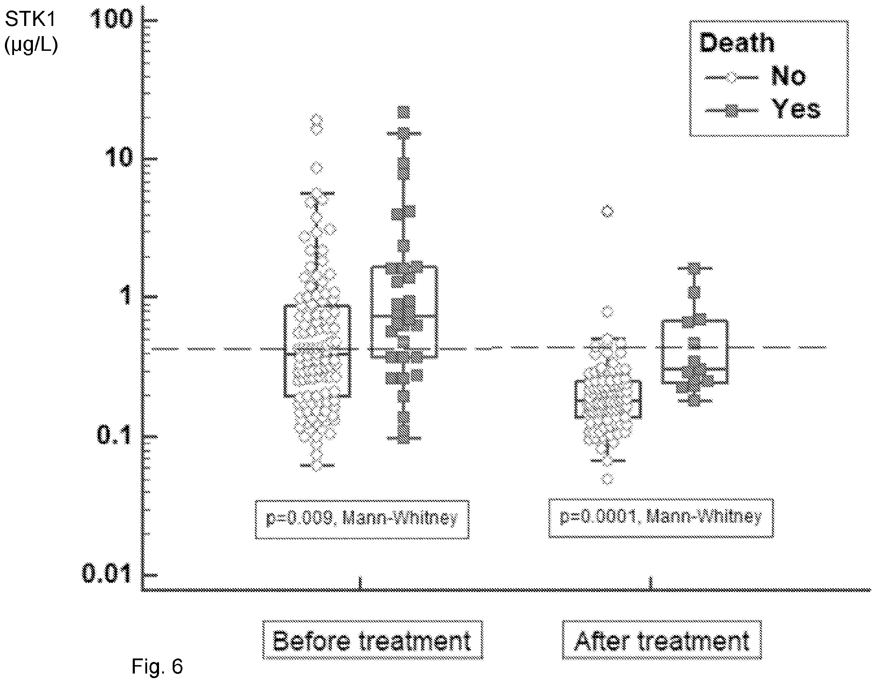

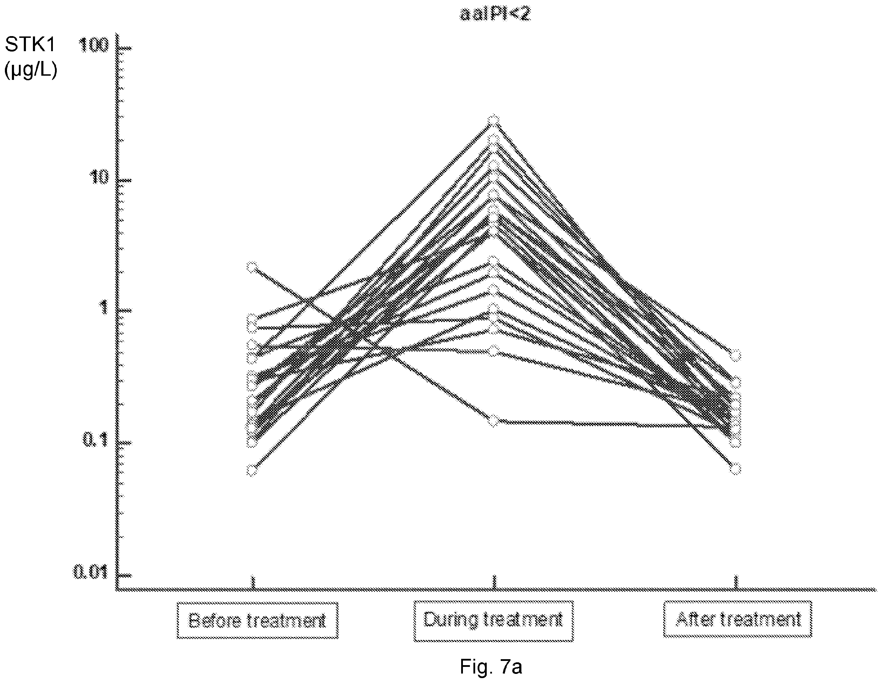

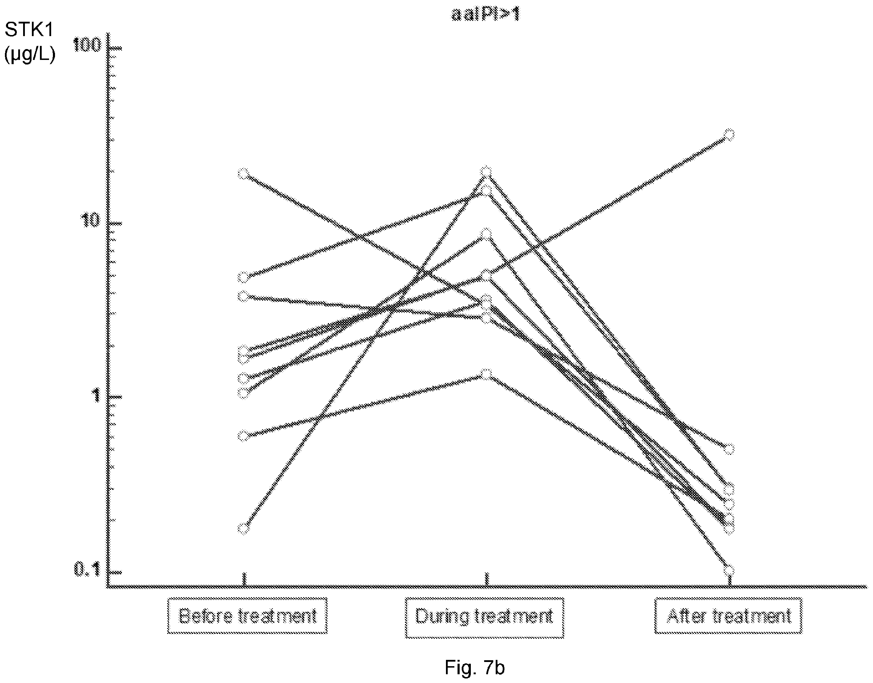

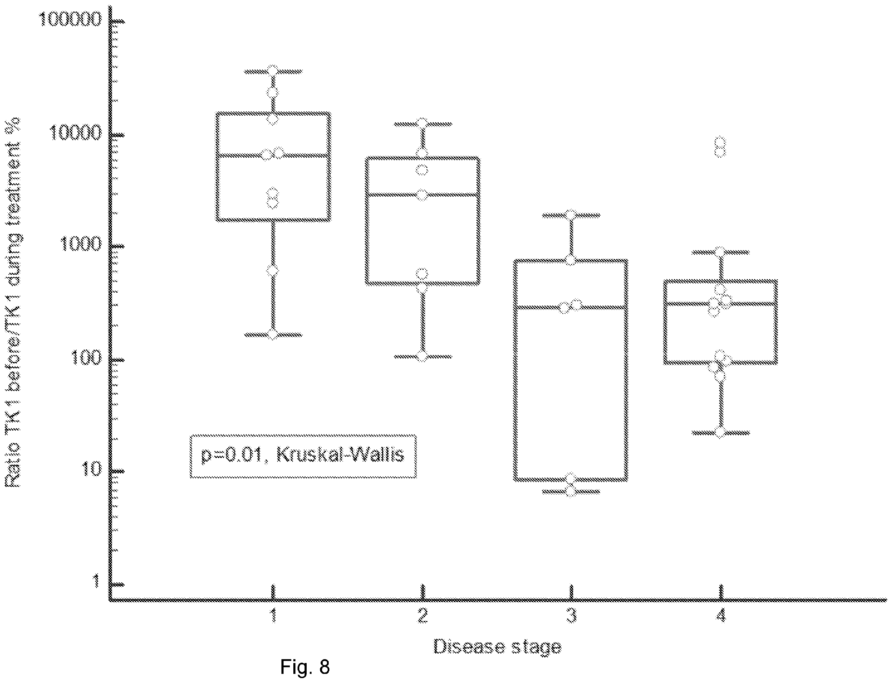

In a fraction of the patients (40/146, 27%), TK1 concentrations were measured during active treatment, after 3 treatment cycles. Overall, the concentrations increased about ten-fold during treatment (p<0.00001) (Fig. 5) to later decline below initial value (Table 3). Patients with disease relapse (n=18) or death from lymphoma (n=12) had significantly higher TK1 concentrations after treatment compared to patients without relapse or death (p=0.002 and p=0.0001 respectively) (Fig. 6). The changes in TK1 concentrations during the treatment in relation to aaIPI are described in Table 3 and Figs. 7a and 7b. The relative change in TK1 concentrations between measurements at diagnosis compared to measurements after the third treatment cycle, was 3832% in stages 1 and 2 as compared to 302% in stages 3 and 4 (p=0.0008). The relative changes in relation to stages are also illustrated in Fig. 8.

**Table 3 - Mann-Whitney U-test was used for statistical comparison between before and respectively, during and after treatment**

| **aaIPI 0 and 1** | | |
|---|---|---|
| Before treatment¹ | 0.33 mg/L (0.18-0.59 mg/L) | N=85 |
| During treatment² | 4.11 mg/L (1.05-7.1 mg/L) | N=27, p<0.0001 |
| After treatment³ | 0.19 mg/L (0.14-0.25 mg/L) | N=68, p<0.0001 |

| **aaIPI 2 and 3** | | |
|---|---|---|
| Before treatment¹ | 0.99 mg/L (0.45-3.17 mg/L) | N=45 |
| During treatment² | 4.97 mg/L (3.14-11.0 mg/L) | N=12, p=0.004 |
| After treatment³ | 0.24 mg/L (0.17-0.31 mg/L) | N=28, p<0.0001 |

| | | |
|---|---|---|
| aalPl: age adjusted International Prognostic Index | | |

According to protocol blood samples were to be obtained at ¹time of diagnosis (or at least before start of treatment), ²after 3 chemotherapy cycles (regardless if treatment cycles were of length 14 or 21 days), and ³3 month and 1 year after treatment ended. TK1 was analyzed in these blood samples.

Table 4 below summaries hazard ratios for relapse in DLBCL patients and calculated p-values using different cut-off values ranging from 0.25 µg/L up to 2.5 µg/L for all patients.

**Table 4 - Hazards ratio for relapse in diffuse large B-cell lymphoma as judged by Kaplan-Meier statistics of TK1 results at diagnosis and before treatment (n=137)**

| **Cut-off** | **Hazards ratio** | **95% CI** | **p-value** |
|---|---|---|---|
| 0.25 µg/L | 1.87 vs 0.53 | 0.92-3.8 vs 0.26-0.71 | 0.09 |
| 0.35 µg/L | 2.60 vs 0.38 | 1.36-5.0 vs 0.20-0.73 | 0.0037 |
| 0.45 µg/L | 3.03 vs 0.33 | 1.58-5.8 vs 0.17-0.63 | 0.0008 |
| 0.60 µg/L | 3.51 vs 0.28 | 1.77-6.9 vs 0.14-0.56 | 0.0003 |
| 0.75 µg/L | 3.27 vs 0.31 | 1.58-6.7 vs 0.15-0.63 | 0.0014 |
| 1.05 µg/L | 3.06 vs 0.33 | 1.36-6.9 vs 0.15-0.74 | 0.0069 |
| 2.5 µg/L | 2.57 vs 0.39 | 0.81-8.1 vs 0.12-1.23 | 0.11 |

Table 5 and Table 6 list the corresponding hazard ratios for relapse in DLBCL patients >66 year (Table 5) or DLBCL patients <67 years (Table 6) and calculated p-values using different cut-off values ranging from 0.25 µg/L up to 2.5 µg/L for all patients.

**Table 5 - Hazards ratio for relapse in diffuse large B-cell lymphoma as judged by Kaplan-Meier statistics of TK1 results at diagnosis and before treatment, age >66 years (n=67)**

| **Cut-off** | **Hazards ratio** | **95% CI** | **p-value** |
|---|---|---|---|
| 0.25 µg/L | 1.97 vs 0.51 | 0.83-4.70 vs 0.21-1.20 | 0.12 |
| 0.35 µg/L | 3.38 vs 0.30 | 1.53-7.45 vs 0.13-0.65 | 0.0025 |
| 0.45 µg/L | 5.51 vs 0.18 | 2.32-13.0 vs 0.07-0.43 | 0.0001 |
| 0.60 µg/L | 5.3 vs 0.19 | 2.16-13.1 vs 0.08-0.46 | 0.0003 |
| 0.75 µg/L | 4.19 vs 0.24 | 1.61-10.8 vs 0.09-0.62 | 0.003 |
| 1.05 µg/L | 4.11 vs 0.24 | 1.31-12.9 vs 0.08-0.76 | 0.01 |
| 2.5 µg/L | 5.71 vs 0.17 | 0.83-39 vs 0.02-1.19 | 0.07 |

**Table 6 - Hazards ratio for relapse in diffuse large B-cell lymphoma as judged by Kaplan-Meier statistics of TK1 results at diagnosis and before treatment, age <67 years (n=68)**

| **Cut-off** | **Hazards ratio** | **95% CI** | **p-value** |
|---|---|---|---|
| 0.25 µg/L | 1.75 vs 0.57 | 0.49-6.18 vs 0.16-2.01 | 0.39 |
| 0.35 µg/L | 2.51 vs 0.40 | 0.78-8.03 vs 0.12-1.27 | 0.12 |
| 0.45 µg/L | 2.73 vs 0.37 | 0.89-8.49 vs 0.12-1.13 | 0.08 |
| 0.60 µg/L | 3.45 vs 0.29 | 1.07-11.1 vs 0.09-0.93 | 0.038 |
| 0.75 µg/L | 3.88 vs 0.26 | 1.12-13.4 vs 0.07-0.89 | 0.032 |
| 1.05 µg/L | 4.63 vs 0.22 | 1.20-17.9 vs 0.05-0.83 | 0.026 |
| 2.5 µg/L | 4.11 vs 0.24 | 0.68-25 vs 0.04-1.48 | 0.12 |

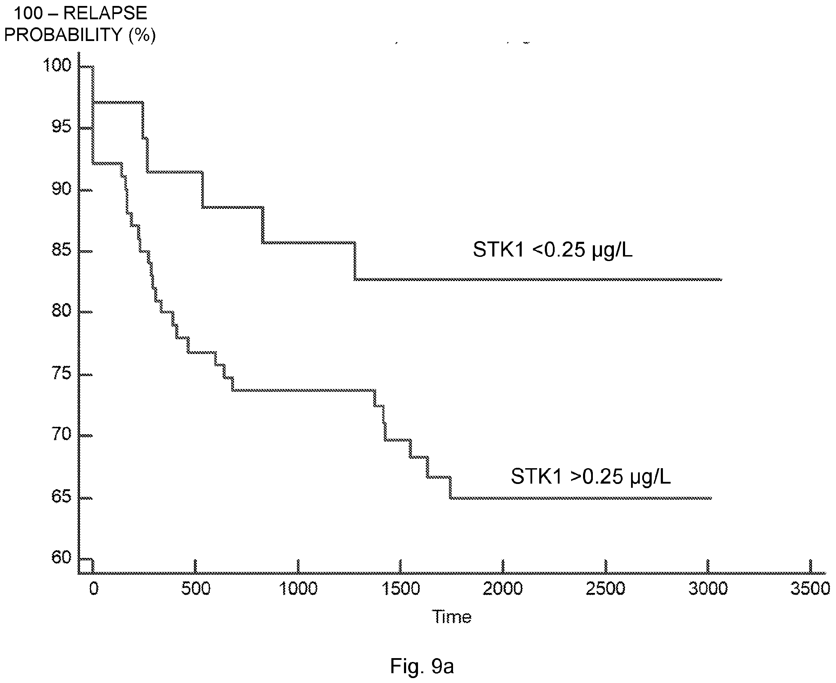

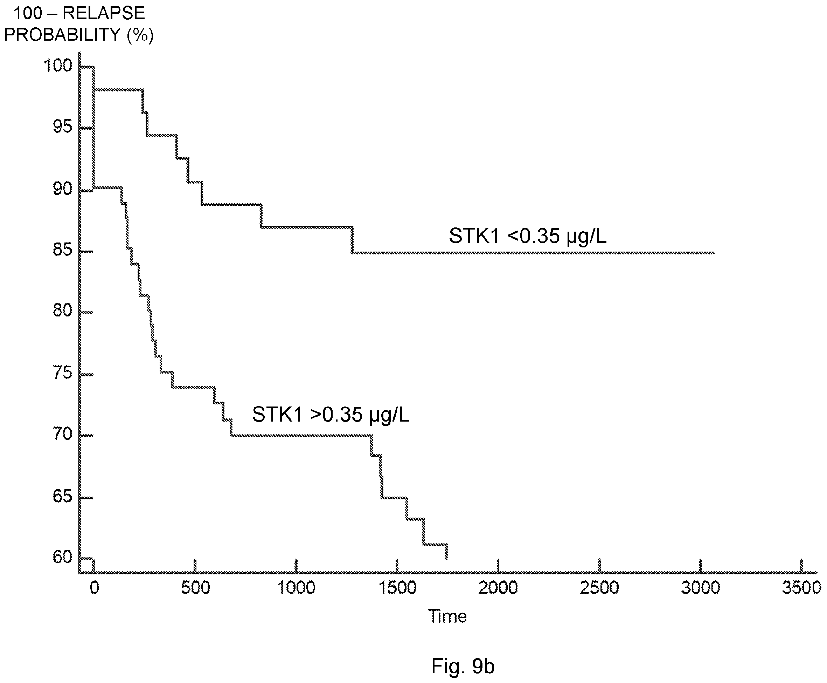

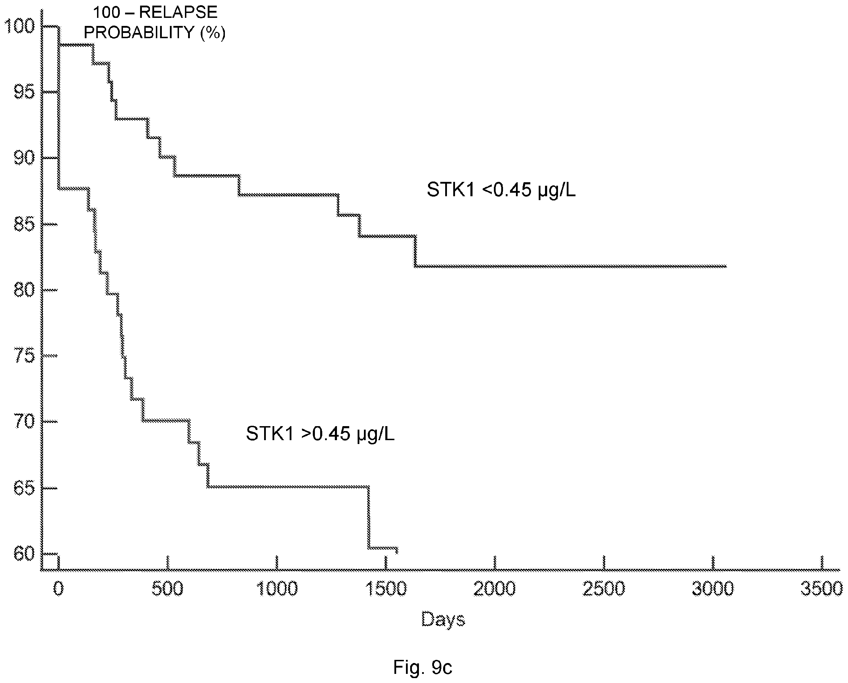

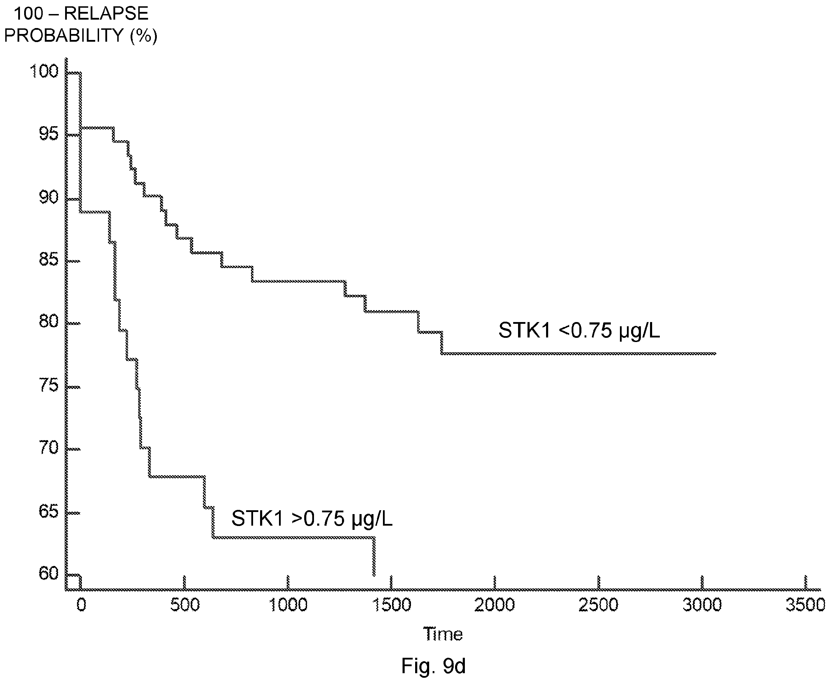

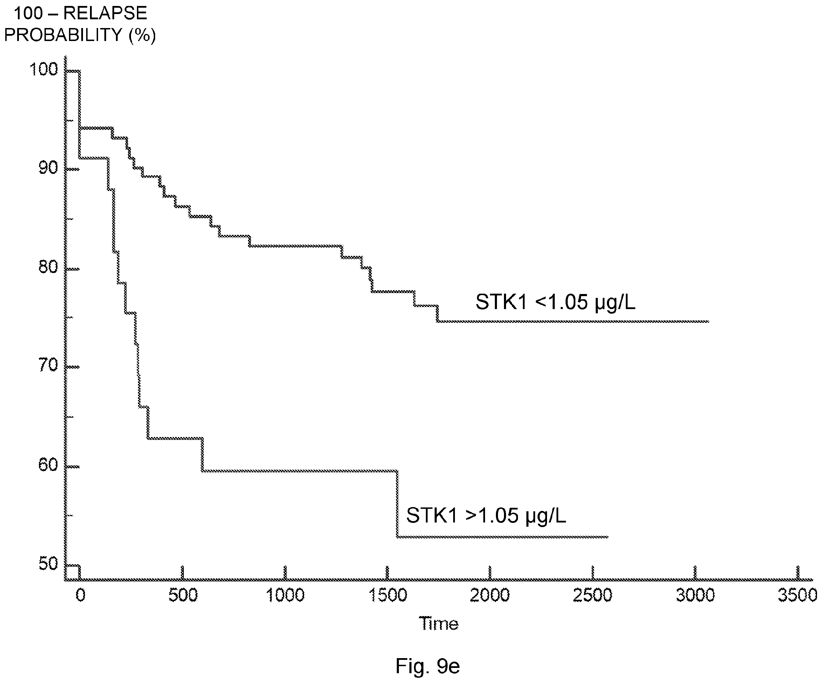

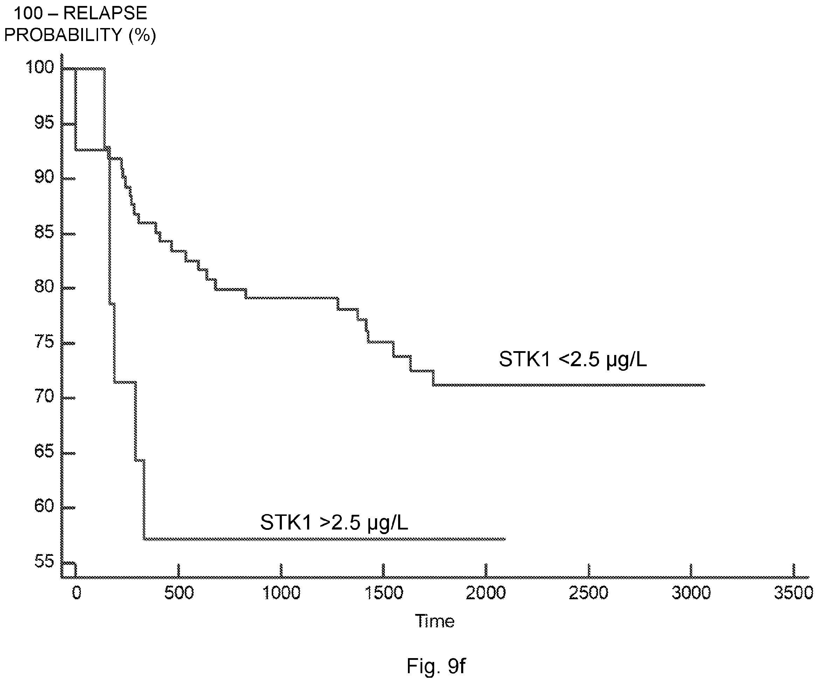

Fig. 9 illustrates Kaplan-Meier plot of probability of disease relapse in relation to TK1 serum concentrations at diagnosis and before treatment in patients with in Diffuse Large B-cell Lymphoma with the cut-off of (Fig. 9a) 0.25 µg/L, (Fig. 9b) 0.35 µg/L, (Fig. 9c) 0.45 µg/L, (Fig. 9d) 0.75 µg/L, (Fig. 9e) 1.05 µg/L and (Fig. 9f) 2.5 µg/L in relation to time of relapse.

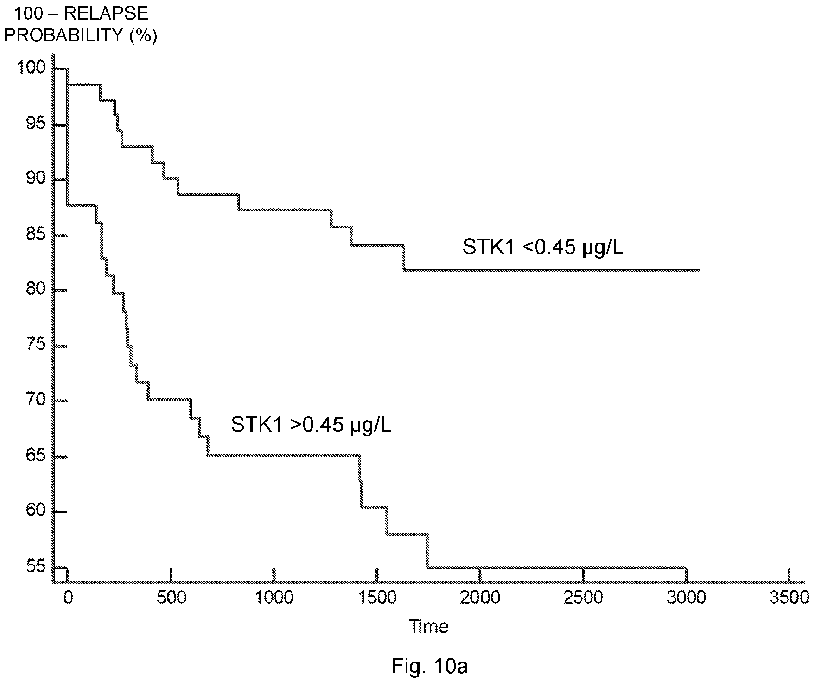

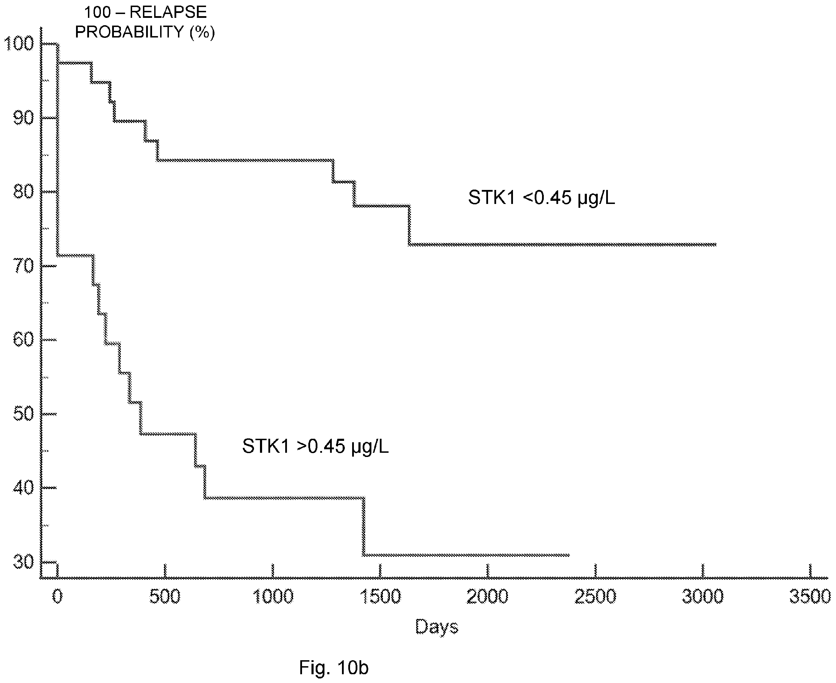

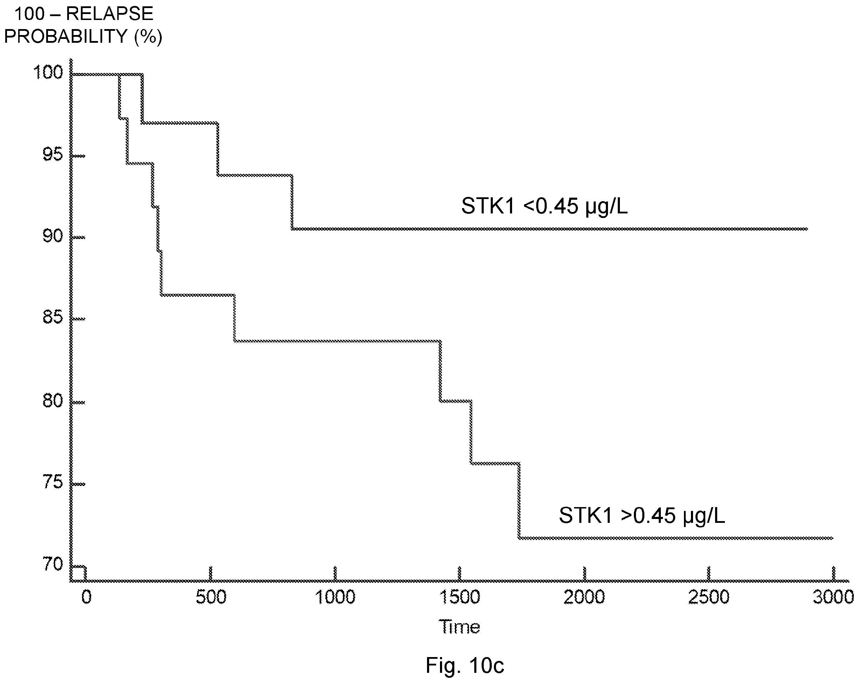

Fig. 10 illustrates Kaplan-Meier plot of probability of disease relapse in relation to TK1 serum concentrations at diagnosis and before treatment in patients with in Diffuse Large B-cell Lymphoma with the cut-off of 0.45 µg/L in relation to time of relapse. Fig. 10a illustrates the results for all patients, Fig. 10b illustrates the results for patients with an age >66 years and Fig. 10c illustrates the results patients with an age<67 years.

### Discussion

This study validated the usefulness of TK1 measurement in patients with lymphoma with very strong relations to disease severity and outcome as seen in Kaplan-Meier curves and associated log rank test as well as in multivariate analyses.

A finding in this study was the increase of serum TK1 during treatment. This increase was significantly higher in patients suffering from disease relapse or death, but also slightly more apparent in patients with lower stages of lymphoma. In contrast, no clear differences were seen when comparing aalPI 0-1 and aalPI 2-3.

We observed in this study a large fraction of treated DLBCL patients with increased serum TK1 and an apparent complete remission of disease. In this study we did not observe any difference in negative events in the group with low response in TK1 but we related this to the overall low number of events (relapse and/or death) among patients with available TK-samples.

There results from Tables 5 and 6 indicated that even an approximately twofold increase in STK1 levels for older patients was correlated with a large increase in the hazard ratios. Furthermore, the hazard ratios were only moderately further increased at higher STK1 levels for older patients. This means that in this patient group, higher STK1 levels are not proportionally related to higher risk for cancer relapse in patients at higher age.

The embodiments described above are to be understood as a few illustrative examples of the present invention. It will be understood by those skilled in the art that various modifications, combinations and changes may be made to the embodiments without departing from the scope of the present invention. In particular, different part solutions in the different embodiments can be combined in other configurations, where technically possible.

## Claims

1. A method for predicting cancer relapse comprising:
determining a level of serum thymidine kinase 1 (STK1) material in a serum sample or a plasma sample from a patient diagnosed with hematological cancer using an antibody or a fragment thereof specifically binding to a serum form of human TK1;
selecting a threshold value based on an age of the patient;
comparing the determined level of STK1 material with the threshold value selected based on the age of the patient; and
predicting cancer relapse of the patient based on the comparison.

2. The method according to claim 1, further comprising estimating a hazard ratio (HR) for the patient based on the comparison, wherein predicting cancer relapse comprises predicting cancer relapse of the patient based on the estimated HR.

3. The method according to claim 1 or 2, wherein predicting cancer relapse comprises predicting a high risk for cancer relapse of the patient if the determined level of STK1 material in the serum sample or plasma sample exceeds the selected threshold value and otherwise predicting a low risk for cancer relapse of the patient.

4. The method according to any of the claims 1 to 3, further comprising:
selecting a first threshold value if the age of the patient is equal to or above a defined age; and
selecting a second, different threshold value if the age of the patient is below the defined age, wherein the second, different threshold value is preferably higher than the first threshold value.

5. The method according to claim 4, wherein the defined age is 67 years.

6. The method according to any of the claims 1 to 5, wherein the hematological cancer is selected from the group consisting of lymphoma, leukemia and multiple myeloma, preferably the hematological cancer is lymphoma, and more preferably the hematological cancer is diffuse large B-cell lymphoma (DLBCL).

7. The method according to any of the claims 1 to 6, wherein determining the level of STK1 material in the serum sample or plasma sample comprises:
contacting the serum sample or plasma sample with the antibody or the fragment thereof; and
measuring an amount of the antibody or the fragment thereof bound to the STK1 material.

8. The method according to claim 7, further comprising correlating the measured amount of the antibody or the fragment thereof bound to the STK1 material to a level of STK1 material, preferably using a pre-defined correlation between measured amount of the antibody or the fragment thereof bound to recombinant human TK1 and a concentration of recombinant human TK1.

9. The method according to any of the claims 1 to 8, wherein determining the level of STK1 material comprises determining, using the antibody or the fragment thereof specifically binding to the serum form of human TK1, the level of STK1 material in the serum sample or plasma sample taken from the patient in connection with diagnosing the patient with hematological cancer or prior to start of treatment of the hematological cancer.

10. The method according to any of the claims 1 to 9, wherein the antibody or the fragment thereof is a monoclonal antibody or a fragment thereof specifically binding to the serum form of human TK1.

11. The method according to claim 10, wherein the monoclonal antibody or the fragment thereof is selected from the group consisting of:
a monoclonal antibody or a fragment thereof having specificity for GEAVAARKLF (SEQ ID NO: 1) of human TK1;
a monoclonal antibody or a fragment thereof having specificity for at least one of NCPVPGKPGE (SEQ ID NO: 2), PVPGKPGEAV (SEQ ID NO: 3) and NCPVPGKPGEAV (SEQ ID NO: 4) of human TK1; and
a monoclonal antibody or a fragment thereof having specificity for a conformation dependent epitope of human TK1.

12. The method according to claim 11, wherein
the monoclonal antibody or the fragment thereof has
a variable heavy (VH) domain complementarity determining region 1 (CDR1) having amino acid sequence SEQ ID NO: 5;
a VH domain CDR2 having amino acid sequence SEQ ID NO: 6;
a VH domain CDR3 having amino acid sequence SEQ ID NO: 7;
a variable light (VL) domain CDR1 having amino acid sequence SEQ ID NO: 8;
a VL domain CDR2 having amino acid sequence SEQ ID NO: 9; and
a VL domain CDR3 having amino acid sequence SEQ ID NO: 10; or
the monoclonal antibody or the fragment thereof has
a variable heavy (VH) domain complementarity determining region 1 (CDR1) having amino acid sequence SEQ ID NO: 5;
a VH domain CDR2 having amino acid sequence SEQ ID NO: 11;
a VH domain CDR3 having amino acid sequence SEQ ID NO: 12;
a variable light (VL) domain CDR1 having amino acid sequence SEQ ID NO: 13;
a VL domain CDR2 having amino acid sequence SEQ ID NO: 9; and
a VL domain CDR3 having amino acid sequence SEQ ID NO: 10; or
the monoclonal antibody or the fragment thereof has
a variable heavy (VH) domain complementarity determining region 1 (CDR1) having amino acid sequence SEQ ID NO: 14;
a VH domain CDR2 having amino acid sequence SEQ ID NO: 15;
a VH domain CDR3 having amino acid sequence SEQ ID NO: 16;
a variable light (VL) domain CDR1 having amino acid sequence SEQ ID NO: 17;
a VL domain CDR2 having amino acid sequence SEQ ID NO: 18; and
a VL domain CDR3 having amino acid sequence SEQ ID NO: 19.

13. The method according to claim 11 or 12, wherein determining the level of STK1 material comprises determining the level of STK1 material in the serum sample or plasma sample using a kit for determining a level of STK1 material in a serum sample or plasma sample comprising:
a first monoclonal antibody or a first fragment thereof having specificity for an epitope selected from the group consisting of:
GEAVAARKLF (SEQ ID NO: 1) of human TK1;
at least one of NCPVPGKPGE (SEQ ID NO: 2), PVPGKPGEAV (SEQ ID NO: 3) and NCPVPGKPGEAV (SEQ ID NO: 4) of human TK1; and
a conformation dependent epitope of human TK1; and
a second monoclonal antibody or a second fragment thereof having specificity for an epitope selected from the group consisting of:
GEAVAARKLF (SEQ ID NO: 1) of human TK1;
at least one of NCPVPGKPGE (SEQ ID NO: 2), PVPGKPGEAV (SEQ ID NO: 3) and NCPVPGKPGEAV (SEQ ID NO: 4) of human TK1; and
a conformation dependent epitope of human TK1, wherein preferably one of the first monoclonal antibody or first fragment thereof and the second monoclonal antibody or second fragment thereof is immobilized to a solid support or intended to be immobilized to the solid support.

14. The method according to claim 13, wherein the kit is an Enzyme-Linked Immunosorbent Assay (ELISA) kit.

## Patentansprüche

1. Verfahren zum Vorhersagen eines Krebsrückfalls, umfassend:
Bestimmen eines Niveaus von Serum-Thymidinkinase-1-(STK1-)Material in einer Serumprobe oder einer Plasmaprobe eines Patienten, bei dem eine hämatologische Krebserkrankung diagnostiziert wurde, unter Verwendung eines Antikörpers oder eines Fragments davon, das spezifisch an eine Serumform von humaner TK1 bindet;
Auswählen eines Schwellenwerts basierend auf dem Alter des Patienten;
Vergleichen des bestimmten Niveaus des STK1-Materials mit dem basierend auf dem Alter des Patienten ausgewählten Schwellenwert; und
Vorhersagen eines Krebsrückfalls des Patienten basierend auf dem Vergleich.

2. Verfahren nach Anspruch 1, ferner umfassend das Abschätzen eines Hazard Ratios (HR) für den Patienten basierend auf dem Vergleich, wobei das Vorhersagen eines Krebsrückfalls das Vorhersagen eines Krebsrückfalls des Patienten basierend auf dem geschätzten HR umfasst.

3. Verfahren nach Anspruch 1 oder 2, wobei das Vorhersagen eines Krebsrückfalls das Vorhersagen eines hohen Risikos für einen Krebsrückfall des Patienten umfasst, wenn das bestimmte Niveau des STK1-Materials in der Serumprobe oder Plasmaprobe den ausgewählten Schwellenwert überschreitet, und anderweitiges Vorhersagen eines niedrigen Risikos für einen Krebsrückfall des Patienten.

4. Verfahren nach einem der Ansprüche 1 bis 3, ferner umfassend:
Auswählen eines ersten Schwellenwerts, wenn das Alter des Patienten gleich oder größer als ein definiertes Alter ist; und Auswählen eines zweiten, unterschiedlichen Schwellenwerts, wenn das Alter des Patienten unter dem definierten Alter liegt, wobei der zweite, unterschiedliche Schwellenwert vorzugsweise höher als der erste Schwellenwert ist.

5. Verfahren nach Anspruch 4, wobei das definierte Alter 67 Jahre beträgt.

6. Verfahren nach einem der Ansprüche 1 bis 5, wobei die hämatologische Krebserkrankung aus der Gruppe ausgewählt ist, bestehend aus Lymphom, Leukämie und multiplem Myelom, wobei die hämatologische Krebserkrankung vorzugsweise ein Lymphom und noch bevorzugter wobei die hämatologische Krebserkankung ein diffus großzelliges B-Zell-Lymphom (DLBCL) ist.

7. Verfahren nach einem der Ansprüche 1 bis 6, wobei das Bestimmen des Niveaus des STK1-Materials in der Serumprobe oder Plasmaprobe umfasst:
Inkontaktbringen der Serumprobe oder Plasmaprobe mit dem Antikörper oder dem Fragment davon; und
Messen einer Menge des Antikörpers oder des Fragments davon, die an das STK1-Material gebunden ist.

8. Verfahren nach Anspruch 7, ferner umfassend das Korrelieren der gemessenen Menge des Antikörpers oder des Fragments davon, die an das STK1-Material gebunden ist, mit einem Niveau des STK1-Materials, vorzugsweise unter Verwendung einer vordefinierten Korrelation zwischen der gemessenen Menge des Antikörpers oder des Fragments davon, die an rekombinante humane TK1 gebunden ist, und einer Konzentration von rekombinanter humaner TK1.

9. Verfahren nach einem der Ansprüche 1 bis 8, wobei das Bestimmen des Niveaus des STK1-Materials das Bestimmen des Niveaus des STK1-Materials in der Serumprobe oder Plasmaprobe, die dem Patienten im Zusammenhang mit der Diagnose der hämatologischen Krebserkrankung oder vor Beginn der Behandlung der hämatologischen Krebserkrankung entnommen wurde, unter Verwendung des Antikörpers oder des Fragments davon, der spezifisch an die Serumform von humaner TK1 bindet, umfasst.

10. Verfahren nach einem der Ansprüche 1 bis 9, wobei der Antikörper oder das Fragment davon ein monoklonaler Antikörper oder ein Fragment davon ist, der spezifisch an die Serumform von humaner TK1 bindet.

11. Verfahren nach Anspruch 10, wobei der monoklonale Antikörper oder das Fragment davon aus der Gruppe ausgewählt ist, bestehend aus:
einem monoklonalen Antikörper oder einem Fragment davon mit Spezifität für GEAVAARKLF (SEQ ID NO: 1) von humaner TK1;
einem monoklonalen Antikörper oder einem Fragment davon mit Spezifität für mindestens eines von NCPVPGKPGE (SEQ ID NO: 2), PVPGKPGEAV (SEQ ID NO: 3) und NCPVPGKPGEAV (SEQ ID NO: 4) von humaner TK1; und
einem monoklonalen Antikörper oder einem Fragment davon mit Spezifität für ein konformationsabhängiges Epitop von humaner TK1.

12. Verfahren nach Anspruch 11, wobei
der monoklonale Antikörper oder das Fragment davon aufweist:
eine komplementaritätsbestimmende Region 1 (CDR1) der variablen schweren (VH) Domäne mit der Aminosäuresequenz SEQ ID NO: 5;
eine VH-Domänen-CDR2 mit der Aminosäuresequenz SEQ ID NO: 6;
eine VH-Domänen-CDR3 mit der Aminosäuresequenz SEQ ID NO: 7;
eine CDR1 der variablen leichten (VL) Domäne mit der Aminosäuresequenz SEQ ID NO: 8;
eine VL-Domänen-CDR2 mit der Aminosäuresequenz SEQ ID NO: 9; und
eine VL-Domänen-CDR3 mit der Aminosäuresequenz SEQ ID NO: ; oder
der monoklonale Antikörper oder das Fragment davon aufweist:
eine komplementaritätsbestimmende Region 1 (CDR1) der variablen schweren (VH) Domäne mit der Aminosäuresequenz SEQ ID NO: 5;
eine VH-Domänen-CDR2 mit der Aminosäuresequenz SEQ ID NO: 11;
eine VH-Domänen-CDR3 mit der Aminosäuresequenz SEQ ID NO: 12;
eine CDR1 der variablen leichten (VL) Domäne mit der Aminosäuresequenz SEQ ID NO: 13;
eine VL-Domänen-CDR2 mit der Aminosäuresequenz SEQ ID NO: 9; und
eine VL-Domänen-CDR3 mit der Aminosäuresequenz SEQ ID NO: ; oder
der monoklonale Antikörper oder das Fragment davon aufweist:
eine komplementaritätsbestimmende Region 1 (CDR1) der variablen schweren (VH) Domäne mit der Aminosäuresequenz SEQ ID NO: 14;
eine VH-Domänen-CDR2 mit der Aminosäuresequenz SEQ ID NO: 15;
eine VH-Domänen-CDR3 mit der Aminosäuresequenz SEQ ID NO: 16;
eine CDR1 der variablen leichten (VL) Domäne mit der Aminosäuresequenz SEQ ID NO: 17;
eine VL-Domänen-CDR2 mit der Aminosäuresequenz SEQ ID NO: 18; und
eine VL-Domänen-CDR3 mit der Aminosäuresequenz SEQ ID NO: 19.

13. Verfahren nach Anspruch 11 oder 12, wobei das Bestimmen des Niveaus des STK1-Materials das Bestimmen des Niveaus des STK1-Materials in der Serumprobe oder Plasmaprobe unter Verwendung eines Kits zum Bestimmen eines Niveaus von STK1-Material in einer Serumprobe oder Plasmaprobe umfasst, umfassend:
einen ersten monoklonalen Antikörper oder ein erstes Fragment davon mit Spezifität für ein Epitop, ausgewählt aus der Gruppe, bestehend aus:
GEAVAARKLF (SEQ ID NO: 1) von humaner TK1;
mindestens einem von NCPVPGKPGE (SEQ ID NO: 2), PVPGKPGEAV (SEQ ID NO: 3) und NCPVPGKPGEAV (SEQ ID NO: 4) von humaner TK1; und
einem konformationsabhängigen Epitop von humaner TK1; und
einen zweiten monoklonalen Antikörper oder ein zweites Fragment davon mit Spezifität für ein Epitop, ausgewählt aus der Gruppe, bestehend aus:
GEAVAARKLF (SEQ ID NO: 1) von humaner TK1;
mindestens einem von NCPVPGKPGE (SEQ ID NO: 2), PVPGKPGEAV (SEQ ID NO: 3) und NCPVPGKPGEAV (SEQ ID NO: 4) von humaner TK1; und
einem konformationsabhängigen Epitop von humaner TK1, wobei vorzugsweise eines des ersten monoklonalen Antikörpers oder des ersten Fragments davon und des zweiten monoklonale Antikörpers oder des zweiten Fragments davon an einen festen Träger immobilisiert ist oder zur Immobilisierung an dem festen Träger vorgesehen ist.

14. Verfahren nach Anspruch 13, wobei das Kit ein Kit eines Enzyme-Linked-Immunosorbent-Assays (ELISA) ist.

## Revendications

1. Procédé de prédiction d'une rechute de cancer comprenant :
la détermination d'un taux de matériel de thymidine kinase 1 sérique (STK1) dans un échantillon de sérum ou un échantillon de plasma provenant d'un patient diagnostiqué avec un cancer hématologique en utilisant un anticorps ou un fragment de celui-ci se liant spécifiquement à une forme sérique de TK1 humaine ;
la sélection d'une valeur de seuil sur la base d'un âge du patient ;
la comparaison du taux déterminé de matériel de STK1 avec la valeur de seuil sélectionnée sur la base de l'âge du patient ; et
la prédiction de la rechute de cancer du patient sur la base de la comparaison.

2. Procédé selon la revendication 1, comprenant en outre l'estimation d'un rapport de risque (HR) pour le patient sur la base de la comparaison, dans lequel la prédiction de la rechute de cancer comprend la prédiction de la rechute de cancer du patient sur la base du HR estimé.

3. Procédé selon la revendication 1 ou 2, dans lequel la prédiction de la rechute de cancer comprend la prédiction d'un risque élevé de rechute de cancer du patient si le taux déterminé de matériel de STK1 dans l'échantillon de sérum ou l'échantillon de plasma dépasse la valeur de seuil sélectionnée et, sinon, la prédiction d'un risque faible de rechute de cancer du patient.

4. Procédé selon l'une quelconque des revendications 1 à 3, comprenant en outre :
la sélection d'une première valeur de seuil si l'âge du patient est égal ou supérieur à un âge défini ; et
la sélection d'une seconde valeur de seuil différente si l'âge du patient est inférieur à l'âge défini, dans lequel la seconde valeur de seuil différente est de préférence supérieure à la première valeur de seuil.

5. Procédé selon la revendication 4, dans lequel l'âge défini est 67 ans.

6. Procédé selon l'une quelconque des revendications 1 à 5, dans lequel le cancer hématologique est choisi dans le groupe constitué par un lymphome, une leucémie et un myélome multiple, de préférence le cancer hématologique est un lymphome, et plus préférablement le cancer hématologique est un lymphome diffus à grandes cellules B (DLBCL).

7. Procédé selon l'une quelconque des revendications 1 à 6, dans lequel la détermination du taux de matériel de STK1 dans l'échantillon de sérum ou l'échantillon de plasma comprend :
la mise en contact de l'échantillon de sérum ou de l'échantillon de plasma avec l'anticorps ou le fragment de celui-ci ; et
la mesure d'une quantité de l'anticorps ou du fragment de celui-ci lié au matériel de STK1.

8. Procédé selon la revendication 7, comprenant en outre la corrélation de la quantité mesurée de l'anticorps ou du fragment de celui-ci lié au matériel de STK1 à un taux de matériel de STK1, de préférence en utilisant une corrélation prédéfinie entre la quantité mesurée de l'anticorps ou du fragment de celui-ci lié à une TK1 humaine recombinante et une concentration de TK1 humaine recombinante.

9. Procédé selon l'une quelconque des revendications 1 à 8, dans lequel la détermination du taux de matériel de STK1 comprend la détermination, en utilisant l'anticorps ou le fragment de celui-ci se liant spécifiquement à la forme sérique de TK1 humaine, du taux de matériel de STK1 dans l'échantillon de sérum ou l'échantillon de plasma prélevé à partir du patient en relation avec le diagnostic du patient avec un cancer hématologique ou avant le début du traitement du cancer hématologique.

10. Procédé selon l'une quelconque des revendications 1 à 9, dans lequel l'anticorps ou le fragment de celui-ci est un anticorps monoclonal ou un fragment de celui-ci se liant spécifiquement à la forme sérique de TK1 humaine.

11. Procédé selon la revendication 10, dans lequel l'anticorps monoclonal ou le fragment de celui-ci est choisi dans le groupe constitué par :
un anticorps monoclonal ou un fragment de celui-ci ayant une spécificité pour GEAVAARKLF (SEQ ID NO : 1) de TK1 humaine ;
un anticorps monoclonal ou un fragment de celui-ci ayant une spécificité pour au moins l'un de NCPVPGKPGE (SEQ ID NO : 2), PVPGKPGEAV (SEQ ID NO : 3) et NCPVPGKPGEAV (SEQ ID NO : 4) de TK1 humaine ; et
un anticorps monoclonal ou un fragment de celui-ci ayant une spécificité pour un épitope dépendant de la conformation de TK1 humaine.

12. Procédé selon la revendication 11, dans lequel
l'anticorps monoclonal ou le fragment de celui-ci possède une région déterminant la complémentarité 1 (CDR1) de domaine lourd variable (VH) ayant la séquence d'acides aminés SEQ ID NO : 5 ;
une CDR2 de domaine VH ayant la séquence d'acides aminés SEQ ID NO : 6 ;
une CDR3 de domaine VH ayant la séquence d'acides aminés SEQ ID NO : 7 ;
une CDR1 de domaine léger variable (VL) ayant la séquence d'acides aminés SEQ ID NO : 8 ;
une CDR2 de domaine VL ayant la séquence d'acides aminés SEQ ID NO : 9 ; et
une CDR3 de domaine VL ayant la séquence d'acides aminés SEQ ID NO : 10 ; ou
l'anticorps monoclonal ou le fragment de celui-ci possède une région déterminant la complémentarité 1 (CDR1) de domaine lourd variable (VH) ayant la séquence d'acides aminés SEQ ID NO : 5 ;
une CDR2 de domaine VH ayant la séquence d'acides aminés SEQ ID NO : 11 ;
une CDR3 de domaine VH ayant la séquence d'acides aminés SEQ ID NO : 12 ;
une CDR1 de domaine léger variable (VL) ayant la séquence d'acides aminés SEQ ID NO : 13 ;
une CDR2 de domaine VL ayant la séquence d'acides aminés SEQ ID NO : 9 ; et
une CDR3 de domaine VL ayant la séquence d'acides aminés SEQ ID NO : 10 ; ou
l'anticorps monoclonal ou le fragment de celui-ci possède une région déterminant la complémentarité 1 (CDR1) de domaine lourd variable (VH) ayant la séquence d'acides aminés SEQ ID NO : 14 ;
une CDR2 de domaine VH ayant la séquence d'acides aminés SEQ ID NO : 15 ;
une CDR3 de domaine VH ayant la séquence d'acides aminés SEQ ID NO : 16 ;
une CDR1 de domaine léger variable (VL) ayant la séquence d'acides aminés SEQ ID NO : 17 ;
une CDR2 de domaine VL ayant la séquence d'acides aminés SEQ ID NO : 18 ; et
une CDR3 de domaine VL ayant la séquence d'acides aminés SEQ ID NO : 19.

13. Procédé selon la revendication 11 ou 12, dans lequel la détermination du taux de matériel de STK1 comprend la détermination du taux de matériel de STK1 dans l'échantillon de sérum ou l'échantillon de plasma en utilisant un kit pour déterminer un taux de matériel de STK1 dans un échantillon de sérum ou un échantillon de plasma comprenant :
un premier anticorps monoclonal ou un premier fragment de celui-ci ayant une spécificité pour un épitope choisi dans le groupe constitué par :
GEAVAARKLF (SEQ ID NO : 1) de TK1 humaine ;
au moins l'un de NCPVPGKPGE (SEQ ID NO : 2), PVPGKPGEAV (SEQ ID NO : 3) et NCPVPGKPGEAV (SEQ ID NO : 4) de TK1 humaine ; et
un épitope dépendant de la conformation de TK1 humaine ; et
un second anticorps monoclonal ou un second fragment de celui-ci ayant une spécificité pour un épitope choisi dans le groupe constitué par :
GEAVAARKLF (SEQ ID NO : 1) de TK1 humaine ;
au moins l'un de NCPVPGKPGE (SEQ ID NO : 2), PVPGKPGEAV (SEQ ID NO : 3) et NCPVPGKPGEAV (SEQ ID NO : 4) de TK1 humaine ; et
un épitope dépendant de la conformation de TK1 humaine, dans lequel de préférence l'un parmi le premier anticorps monoclonal ou le premier fragment de celui-ci et le second anticorps monoclonal ou le second fragment de celui-ci est immobilisé sur un support solide ou est destiné à être immobilisé sur le support solide.

14. Procédé selon la revendication 13, dans lequel le kit est un kit de dosage d'immunoabsorption enzymatique (ELISA).
